(19) **Europäisches Patentamt**

**European Patent Office**

**Office européen des brevets**

(11) Publication number : **0 387 914 B1**

(12) **EUROPEAN PATENT SPECIFICATION**

(45) Date of publication of patent specification :
04.08.93 Bulletin 93/31

(21) Application number : 90105189.6

(22) Date of filing : 18.10.85

(51) Int. Cl.⁵ : **C07K 15/04, A61K 39/21,**
**C12N 15/49, C07K 1/00,**
**C07K 3/04, G01N 33/569**

(54) Envelope antigens of the human immuno-deficiency virus, and their applications.

(30) Priority : **18.10.84 FR 8416013**
**16.11.84 GB 8429099**
**21.01.85 GB 8501473**

(43) Date of publication of application :
**19.09.90 Bulletin 90/38**

(60) Publication number of the earlier application in
accordance with Art. 76 EPC : **0 201 540**

(45) Publication of the grant of the patent :
**04.08.93 Bulletin 93/31**

(84) Designated Contracting States :
**AT BE CH DE FR GB IT LI LU NL SE**

(56) References cited :
SCIENCE, vol. 225, 20th July 1984, pages
321-323; V.S. KALYANARAMAN et al.:
"Antibodies to the core protein of lym-
phadenopathy. Associated virus (LAV) in pa-
tients with AIDS"
SCIENCE, vol. 224, no. 4, 4th May 1984, pages
503-505; J. SCHÜPBACH et al.: "Serological
analysis of a subgroup of human T-lymphotropic retroviruses (HTLV-III) associated with
AIDS"
CELL, vol. 41, no. 3, July 1985, pages 979-986,
MIT; R. CROWL et al.: "HTLV-III env gene
products synthesized in E. coli are recognized
by antibodies present in the sera of AIDS
patients"
NATURE, vol. 313, no. 6000, January 1985,
pages 277-284, London, GB; L. RATNER et al.:
"Complete nucleotide sequence of the AIDS
virus, HTLV-III"
VIROLOGY, vol. 144, no. 1, 1985, pages
283-289, Academic Press, Inc.; L. MONTAG-
NIER et al.: "Identification and antigenicity of
the major envelope glycoprotein of lym-
phadenopathy-associated virus"
PNAS USA Vol. 78(6), 3403-3407

(73) Proprietor : **INSTITUT PASTEUR**
**25-28, rue du Docteur Roux**
**F-75724 Paris Cédex 15 (FR)**
Proprietor : **CENTRE NATIONAL DE LA**
**RECHERCHE SCIENTIFIQUE**
**15, quai Anatole France**
**F-75007 Paris (FR)**

(72) Inventor : **Mongagnier, Luc**
**21, rue de Malabry**
**F-92350 LE-PLESSIS-ROBINSON (FR)**
Inventor : **Krust, Bernard**
**7, rue de Madagascar**
**F-75012 PARIS (FR)**
Inventor : **Chamaret, Solange**
**324, rue Lecourbe**
**F-75015 PARIS (FR)**
Inventor : **Clavel, François**
**83, rue de l'Assomption**
**F-75016 PARIS (FR)**
Inventor : **Chermann, Jean-Claude**
**8, Les Ombrées 1/Route de la Treille**
**F-13011 MARSEILLE (FR)**
Inventor : **Barre-Sinoussi, Françoise**
**50, rue d'Erevan**
**F-92310 ISSY-LES-MOULINEAUX (FR)**
Inventor : **Alizon, Marc**
**71, rue Cardinal Lemoine**
**F-75005 PARIS (FR)**
Inventor : **Sonigo, Pierre**
**23, rue Gutenberg**
**F-75015 PARIS (FR)**
Inventor : **Stewart, Cole**
**6 A rue du Sud**
**F-92140 CLAMART (FR)**
Inventor : **Danos, Olivier**
**3, avenue Lyautey**
**F-92380 GARCHES (FR)**
Inventor : **Wain-Hobson, Simon**
**3, rue Jean de la Fontaine**
**F-78180 MONTIGNY LES BRETONNEUX (FR)**

(74) Representative : **Gutmann, Ernest et al**
**S.C. Ernest Gutmann - Yves Plasseraud 67,**
**boulevard Haussmann**
**F-75008 Paris (FR)**

## Description

The present invention relates to antigens, particularly in a purified form, of the virus of lymphadenopathies (denoted below by the abbreviation LAS) and of the acquired immuno-deficiency syndrome (denoted below by the abbreviation AIDS), to a process for producing these antigens, particularly antigens of the envelopes of these viruses. The invention also relates to polypeptides, whether glycosylated or not, encoded by said DNA sequences.

The causative agent of LAS or AIDS, a retrovirus, has been identified by F. BARRE-SINOUSSI et al, Science, 220, 868 (1983). It has the following characteristics. It is T-lymphotropic; its preferred target is constituted by Leu 3 cells (or T4 lymphocytes) ; it has reverse transcriptase activity necessitating the presence of Mg + and exhibits strong affinity for poly(adenylate-oligodeoxy-thymidylate)(poly(A)-oligo(dT)12-18). It has a density of 1.16-1.17 in a sucrose gradient, an average diameter of 139 nanometers; and a nucleus having an average diameter of 41 nanometers. Antigens of said virus, particularly a protein p25, are recognised immunologically by antibodies contained in sera taken up from patients afflicted with LAS or AIDS. The p25 protein, which is a core protein, is not recognised immunologically by antibodies to the p24 protein of the HTLVI and II viruses. The virus is also free of a p19 protein which is immunologically cross-reactive with the p19 proteins of HTLVI and HTLVII.

Retroviruses of this type (sometimes denoted by the generic abbreviation LAV) have been filed in the National Collection of Micro-organism Cultures of the INSTITUT PASTEUR of Paris, under numbers I-232, I-240 and I-241. Virus strains similar to LAV in all respects from the morphological and immunological point of view have been isolated in other laboratories. Reference is made by way of examples to the retrovirus strains named HTLV-III isolated by R.C. GALLO et al., Science, 224, 500 (1984) and by M.G. SARNGADHARAN et al., Science 224, 506 (1984) respectively and to the retrovirus isolated by M. JAY LEVY et al., Science, 225, 840-842 (1984), which virus was designated ARV. For the ease of language the last mentioned viruses, as well as others which have equivalent morphological and immunological properties, will be designated hereafter under the generic designation "LAV". Reference is also made to European patent application filed 14 September 1984, with the priority of British patent application number 83 24800 filed 15 september 1983 as regards a more detailed description of the LAV retroviruses or the like and of the uses to which extracts of these viruses give rise.

Initially the core antigens were the main antigens of the virus lysates or extracts which were recognised by sera of patients infected with AIDS or LAS, in the test systems which had then been used. A p42 protein, presented as consisting of an envelope protein, had been detected too. In the same manner GALLO et al disclosed a p41 protein which was also deemed to be a possible component of the virus envelope.

Processes for obtaining a LAV virus have also been described. Reference may be made particularly to the article already mentioned of F. BARRE-SINOUSSI et al., as regards the preparation of the virus in T lymphocyte cultures derived either from blood, or from the umbilical cord, or also from bone marrow cells of adult donors in good health. This process comprises particularly the following essential steps :
- producing a viral infection of these T lymphocytes, after activation by a lectin mitogen, with a viral suspension derived from a crude supernatant liquor of lymphocytes producing the virus (initially obtained from a patient infected with AIDS or LAS),
- culturing cells infected with TCGF, in the presence of anti-$\alpha$-interferon sheep serum,
- effecting purification of the virus produced (production starts generally between the 9th and the 15th day following infection and lasts from 10 to 15 days), which purification comprises precipitating the virus in polyethyleneglycol in order to produce a first concentration of the virus, then centrifuging the preparation obtained in a 20-60 % sucrose gradient or in an isotonic gradient of metrizanide (sold under the trade mark NYCODENZ® by NYEGAARD, Oslo) and recovering the virus with the band having a density of 1.16-1.17 in the sucrose gradient or of 1.10-1.11 in the NYCODENZ gradient.

The LAV virus may also be produced from permanent cell lines of type T, such as the CEM line, or from B lymphoblastoid cell lines, such as obtained by the transformation of the lymphocytes derived from a healthy donor with the Epstein-Barr virus, for instance as disclosed in EP-A-0165120. The permanent cell lines obtained produce continuously a virus (designated as LAV-B in the case of the B lymphoblastoïd cell lines) which possesses the essential antigenic and morphological features of the LAV viruses (except that it is collected in a density band sometimes slightly higher than in the preceding case (particularly 1.18) in sucrose. The final purification of the virus can also be carried out in a NYCODENZ gradient.

Reference is made to the article of Schüpbach et al, Science, vol. 224, pages 503-505, 4 May 1984. This article discloses a work with respect to different antigens of the HTLV-III retrovirus.

Results are presented including a reference to an antigen having migrated on an electrophoresis gel at a migration distance corresponding to a molecular weight of approximately 110,000.

Schüpbach et al mentioned that this antigen was detected in a virus preparation but was below limit of detection in the cells.

A method for cloning DNA sequences hybridizable with the genomic RNA of LAV has already been disclosed in EP-A-178978. Reference is hereafter made to that application as concerns the subject matter in common with the further improvements to the invention disclosed herein.

The invention aims at providing purified unaltered virus forms (or viruses less altered by the purification procedures resorted to) and processes for obtaining said unaltered purified viruses.

The present invention further aims at providing additional new means which should not only also be useful for the detection of LAV or related viruses (hereafter more generally referred to as "LAV viruses"), but also have more versatility, particularly in detecting specific parts of the genomic DNA of said viruses whose expression products are not always directly detectable by immunological methods. The present invention further aims at providing polypeptides containing sequences in common with polypeptides comprising antigenic determinants included in the proteins encoded and expressed by the LAV genome occurring in nature. An additional object of the invention is to further provide means for the detection of proteins related to LAV virus, particularly for the diagnosis of AIDS or pre-AIDS or, to the contrary, for the detection of antibodies against the LAV virus or proteins related therewith, particularly in patients afflicted with AIDS or pre-AIDS or more generally in asymptomatic carriers and in blood-related products. Finally the invention also aims at providing immunogenic polypeptides, and more particularly protective polypeptides for use in the preparation of vaccine compositions against AIDS or related syndromes.

The present invention relates to additional DNA fragments, hybridizable with the genomic RNA of LAV as they will be disclosed hereafter, as well as with additional cDNA variants corresponding to the whole genomes of LAV viruses. It further relates to DNA recombinants containing said DNAs or cDNA fragments.

An unaltered purified LAV retrovirus distinguishes from those which have been defined above, in that it includes an amount of one or several envelope antigens, sufficient to be visualized when the virus is labelled with $^{35}$S-cysteine, free of unlabelled cysteine in a proportion of 200 microcuries per ml of medium. These antigens, among which particularly glycoproteins, are recognised selectively in vitro by sera of patients affected with AIDs or LAS or by the sera of asymptomatic carriers of the virus.

A preferred antigen according to the preceding definition obtainable from a lysate of this virus (or by gentle scouring of the envelope of the virus) is a glycoprotein having a molecular weight of the order of 110,000 daltons, as determined by its migration distance in comparison with the distances of migrations, in a same migration system, of standard proteins having known molecular weights. Particularly comparative measurements were made on a 12.5 % polyacrylamide gel under a voltage of 18 V for 18 hours, upon using the following standard proteins (marketed by AMERSHAM) :

- lysozyme-($^{14}$C)-methyl (MW: 14,300),
- carbon dioxide-($^{14}$C)-methyl (MW: 30,000),
- ovalbumin-($^{14}$C)-methyl (MW: 46,000),
- bovin albumin serum ($^{14}$C)-methyl (MW: 69,000),
- phosphorylase b-($^{14}$C)-methyl (MW: 92,500),
- myosine-($^{14}$C)-methyl (MW: 200,000).

The invention relates also to the antigens themselves, particularly that of molecular weight of about 110,000-120,000, which possess also the capability of being recognised by sera of patients suffering from AIDS or LAS or by sera of persons who have been exposed to LAV viruses or those analogous with the latter. These antigens have also the characteristic of forming complexes with concanavalin A, said complex being dissociatable in the presence of O-methyl-$\alpha$-D-mannopyranoside. The antigens according to the invention can also bind to other lectins for example those known under the name "LENTYL-LECTIN". The preferred antigen according to the invention, of molecular weight 110,000, is also sensitive to the action of endoglycosidases. This action is manifested by the production from the antigen of molecular weight 110,000 of a protein having a molecular weight of the order of 90,000, the latter being separable for example by immunoprecipitation or by separation employing the differences in molecular weights (migrations differentiated on gel).

Preferred antigens of the invention are constituted by glycoproteins.

The invention relates also to the process for producing the viruses according to the invention. This process distinguishes essentially from those recalled above at the level of the final purification operation. In particular, the purification step of the process according to the invention is no longer carried out in gradients, but involves the performance of differential centrifugations effected directly on the supernatants of the culture media of the producing cells. These centrifugation operations comprise particularly a first centrifugation at an angular centrifugation velocity, particularly of 10,000 rpm, enabling the removal of nonviral constituents, more particularly of cellular constituents, then a second centrifugation at higher angular velocity, particularly at 45,000 rpm, to obtain the precipitation of the virus itself. In preferred embodiments, the first centrifugation at 10,000 rpm, is maintained for 10 minutes and the second at 45,000 rpm, for 20 minutes. These are, of course, only indicative values, it being understood that it remains within the ability of the specialist to modify the centrifugation conditions, to provide for the separation of the cellular constituents and of the viral constituents.

This modification of the purification process results in the production of viral preparations from which the antigen mentioned can then be isolated more easily, than from virus preparations purified by the previous methods. In any event, the viruses finally obtained by the process of the present invention are more easely recognised by sera of patients or of persons who have been exposed to the LAV virus or to morphologically and antigenically similar strains.

The antigens according to the invention can themselves be obtained from the above disclosed viruses, by lysis (or other suitable processing) of the latter in the presence of any suitable detergent and by recovery and separation of the antigens released. Advantageously, the lysis of the virus is effected in the presence of aprotinin or of any other agent suitable for inhibiting the action of proteases. The separation of the antigens according to the invention can then be carried out by any method known in itself ; for example, it is possible to proceed with a separation of the proteins by employing their respectively different migrations in a predetermined gel, the protein sought being then isolated from the zone of the gel in which it would normally be found in an electrophoresis operation under well determined conditions, having regard to its molecular weight. The antigens according to the invention can however be separated from the lysate of the abovesaid viruses, due to their affinity for lectins, in particular concanavaline A or lentyl-lectin. The lectin used is preferably immobilised on a solid support, such as the cross linked polymer derived from agarose and marketed under the trade mark SEPHAROSE. After washing of the fixed antigens with a suitable buffer, the antigens can be eluted in any suitable manner, particularly by resorting to a O-methyl-$\alpha$-D-mannopyranoside in solution.

A more thorough purification of these antigens can be performed by immunoprecipitation with the sera of patients known to possess antibodies effective against said protein, with concentrated antibody preparations (polyclonal antibodies) or again with monoclonal antibodies, more particularly directed against the antigen according to the invention, in particular that having the molecular weight of 110,000, denoted below by the abbreviation gp110.

Additional characteristics of the invention will appear also in the course of the description, which follows, of the isolation of a virus according to the invention and of antigens, particularly an envelope antigen of the virus. Reference will be made to the drawings in which :

Figure 1 is derived from a photographic reproduction of gel strips which have been used to carry out electrophoreses of lysate extracts of T lymphocytes, respectively infected and uninfected (controls) by a LAV suspension.

Figures 2 and 3 are the restriction map of a complete LAV genome (clone $\lambda$J19) or of fragments thereof.

Figures 4a to 4e are the complete sequence of a LAV viral genome.

Figures 5 and 6 show diagrammatically parts of the three possible reading phases of LAV genomic RNA, including the open reading frames (ORF) apparent in each of said reading phases.

## I - PRODUCTION OF THE VIRUS AND OF ANTIGENS

T lymphocytes, derived from a healthy donor and infected with LAV1, under the conditions described by F. BARRE-SINOUSSI et Coll., on CEM cells derived from a patient afflicted with leukemia and also infected in vitro with LAV1, were kept under cultivation in a medium containing 200 microcuries of $^{35}$S-cystein and devoid of unlabelled cystein. The infected lymphocytes were cultured in a non denaturing medium to prevent the degradation of the antigen sought. The supernatant liquor from the culture medium was then subjected to a first centrifugation at 10,000 rpm for 10 minutes to remove the non viral components, then to a second centrifugation at 45,000 rpm for 20 minutes for sedimenting the virus. The virus pellet was then lysed by detergent in the presence of aprotinin (5 %) particularly under the conditions described in the article of F. BARRE-SINOUSSI et Coll.

The same operation was repeated on lymphocytes taken up from a healthy donor as control.

The various lysates were then immuno-precipitated by sera of patients suffering from AIDS or LAS. Sera originating from healthy donors or of donors infected with other diseases were immunoprecipitated too. The media were then subjected to electrophoreses in a SDS-polyacrylamide gel.

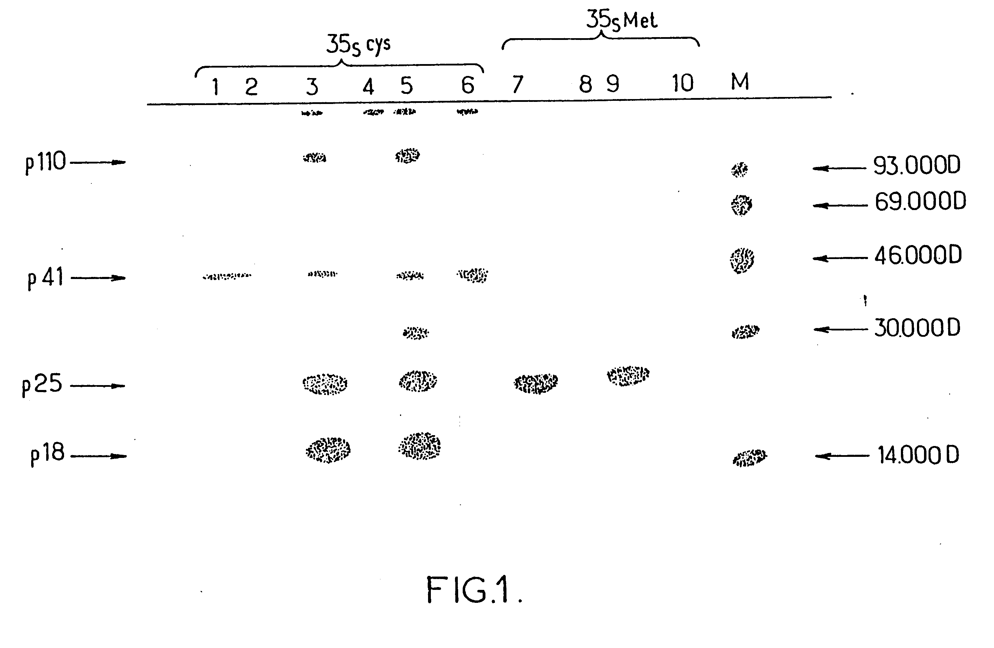

The results are indicated in figure 1. The gel strips numbered from 1 to 6 were obtained from preparations labelled by $^{35}$S-cysteine. The strips numbered 7 to 10 show results observed on infected or uninfected lymphocyte preparations labelled with $^{35}$S-methionine. Finally the strip M corresponds to the migration distances of the standard proteins identified above, whose molecular weights are recalled in the right hand portion of the figure.

The references to the labelled viral proteins appear on the left handside of the figure.

It is noted that columns 7 to 10 show the specific protein p25 of LAV, labelled with $^{35}$S-methionin. The same

protein is absent on strips 8 to 10 corresponding to results obtained with a preparation originating from healthy lymphocytes.

Columns 3 and 5 correspond to the results which have been observed on preparations obtained from lymphocytes infected and labelled with $^{35}$S-cystein. The proteins p25 and p18, the characteristic core proteins of LAV, and the glycoprotein gp110, also specific of LAV, were also present. Images corresponding to a protein p41 (molecular weight of the order of 41,000) appeared in the various preparations, although less distinctly.

The virus according to the invention and the antigen according to the invention can be either precipitated by lectins, particularly concanavaline A, or fixed to a SEPHAROSE®-concanavalin A column. Particularly the purification of the envelope glycoproteins can be carried out as follows. This fixation can particularly be carried out by contacting a lysate of the LAV virus dissolved in a suitable buffer with concanavalin-A bound to SEPHAROSE®. A suitable buffer has the following composition :

Tris 10 mM
NaCl 0.15 M
CaCl$_2$ 1 mM
MgCl$_2$ 1 mM
Detergent marketed under the trade mark TRITON 1 %
pH 7.4

When the fixation has been achieved, the SEPHAROSE®-concanavalin A is washed with a buffer of the same composition, except that the TRITON® concentration is lowered to 0.1 %. The elution is then effected with an 0.2 M O-methyl-$\alpha$-D-mannopyranoside solution in the washing buffer.

The protein may be further concentrated by immuno-precipitation with antibodies contained in the sera of patients infected with AIDS virus or with polyclonal antibodies obtained from a serum derived from an animal previously immunised against the "unaltered" virus according to the invention or the abovesaid glyco-protein. The protein can then be recovered by dissociation of the complex by a solution having an adequate content of ionic salt. Preferably the antibody preparation is itself immobilised in a manner known in itself on an insoluble support, for instance of the SEPHAROSE®B type.

It is also possible to resort to monoclonal antibodies secreted by hybridomas previously prepared against gp 110. These monoclonal antibodies, as well as the hybridomas which produce them, also form part of the invention.

A technique for producing and selecting monoclonal antibodies directed against the gp110 glycoprotein is described below.

## Immunisation of the mice

Groups of Balb/c mice from 6 to 8 weeks old mice were used. One group receives the virus carrying the abovesaid glycoprotein, another a purified glycoprotein gp110. The immunisation procedure, identical for all mice, comprises injecting 10 mg of the antigenic preparation in the presence of Freund's complete adjuvant at day 0, then again but in the presence of Freund incomplete adjuvant at day 14 and without adjuvant at days 28 and 42. The three first injections are made intraperitoneally, the fourth intravenously.

## Fusion and culture of the hybrids

The non secreting myeloma variant 5.53 P3 x 63 Ag8, resistant to azaguanine, itself derived from the MOPC-21 cell-line, is used. Fusion with immunised mouse splenocytes is carried out in the presence of polyethylene-glycol 4000 by the technique of FAZEKAS de st-GROTH and SCHEIDEGGER on the 45th day. The selection of the hybrids in RPMI 16-40 "HAT" medium is carried out in plates having 24 wells (known under the designation COSTAR) by resorting to the same culture techniques.

The hybridomas producing antibodies of adequate specificity are then cloned in plates having 96 wells, in the presence of a "feeder" layer of syngeneic thymocytes. The producing clones thus selected are then expanded in 24 well plates, still in the presence of thymocytes. When the confluence appears in one of the wells, the clone is injected intraperitoneally into a balb/c mouse which had received an injection of PRISTANE 8 days previously and/or is kept in liquid culture.

## Demonstration of the anti-LAV antibodies

Five different techniques enable characterisation of the clones producing antibodies of suitable specificity. In a first stage, the hybrids producing antibodies are determined by an ELISA test revealing mouse immunoglobulins in the supernatant liquors. From this first selection, supernatants are sought which have antibodies directed against viral constituents by means of an ELISA test revealing anti-LAV antibodies, or by immunofluorescence on the virus producing human cells. Finally the supernatant liquors are analysed by radio-immuno-precipitation of virus labelled with cystein and by the Western-Blot technique on a viral preparation which permit the determination of the specificities of these anti-LAV antibodies.

RESULTS

Cells obtained from the various fusions are placed under culture in 648 wells. Their microscopic examination shows that the majority of these wells contain a single hybrid clone capable of growing in a "HAT" selective medium. More than 50 % among them produce antibodies giving rise to a positive response under ELISA antivirus antibody examination. The most representative fusions are tested by the Western-Blot technique and several of them are subcloned, taking into account their respective specificities reactivities in antivirus antibody ELISA and their behaviours under the culturing conditions. Those hybrids which are more particularly selected are those which produce antibodies which selectively recognise the viral glycoprotein gp110 having a molecular weight of about 110 KD. All the sub clonings give rise to clones producing antibodies which, after expression, are injected into syngenic mice. Analysis of the specificities of the antibodies present in the different ascites fluids confirm the specificity of the antibodies of said ascites with respect to gp110.

The monoclonal antibodies obtained can themselves be employed to purify proteins containing an antigenic site also contained in gp110. The invention relates therefore also to these processes of purification as such. This process is advantageously applied to virus lysates or T lymphocyte lysates or other cells producing LAV or the like, when care has been taken to avoid the uncontrolled separation of gp110 during the purification procedure of the virus, prior to lysys thereof. Needless to say that the process can also be applied to any solution containing gp110 or a protein, polypeptide or glycoprotein comprising an antigenic site normally carried by the envelope protein and recognised by the monoclonal antibody. For practising this process, the monoclonal antibodies are advantageously immobilised on a solid support, preferably adapted to affinity chromatography operations. For example, these monoclonal antibodies are fixed to an agarose lattice with three-dimensional cross-linking, marketed under the trade mark SEPHAROSE by the Swedish company PHARMACIA A.G., for example by the cyanogen bromide method.

The invention therefore also relates to a process for separating the antigens concerned, which process comprises contacting a mixture of antigens, including those of interest (for instance a virus lysate or extract), with an affinity column bearing the abovesaid monoclonal antibodies, to selectively fix polypeptides, proteins or glycoproteins selectively recognized by said monoclonal antibodies, recovering the latter by dissociation of the antigen-antibody complex by means of a suitable buffer, particularly a solution of adequate ionic strength, for example of a salt, preferably ammonium acetate (which leaves no residue upon freeze drying of the preparation) or a solution acidified to a pH 2-4 or to a glycine buffer at the same pH and recovering the eluted polypeptides, proteins or glycoproteins.

It is self-evident that the invention relates also to polypeptide fragments having lower molecular weights and carrying antigenic sites recognizable by the same monoclonal antibodies. It is clear to the specialist that the availability of monoclonal antibodies recognizing the gp110 glycoprotein gives also access to smaller peptide sequences or fragments containing the common antigenic site or epitope. Fragments of smaller sizes may be obtained by resorting to known techniques. For instance such a method comprises cleaving the original larger polypeptide by enzymes capable of cleaving it at specific sites. By way of examples of such proteins, may be mentioned the enzyme of Staphylococcyus aureus V8, $\alpha$-chymotrypsin, "mouse sub-maxillary gland protease" marketed by the BOEHRINGER company, Vibrio alginolyticus chemovar iophagus collagenase, which specifically recognises said peptides Gly-Pro and Gly-Ala, etc..

It is also possible to obtain polypeptides or fragments of envelope antigens of the virus, by cloning fragments excised from a cDNA constructed from genomes of LAV variants.

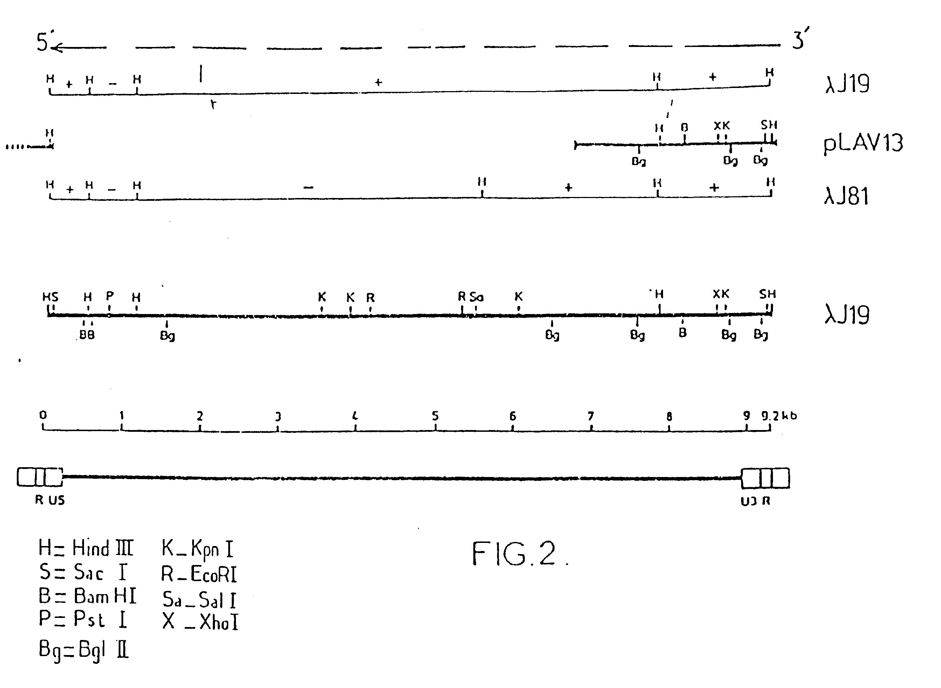

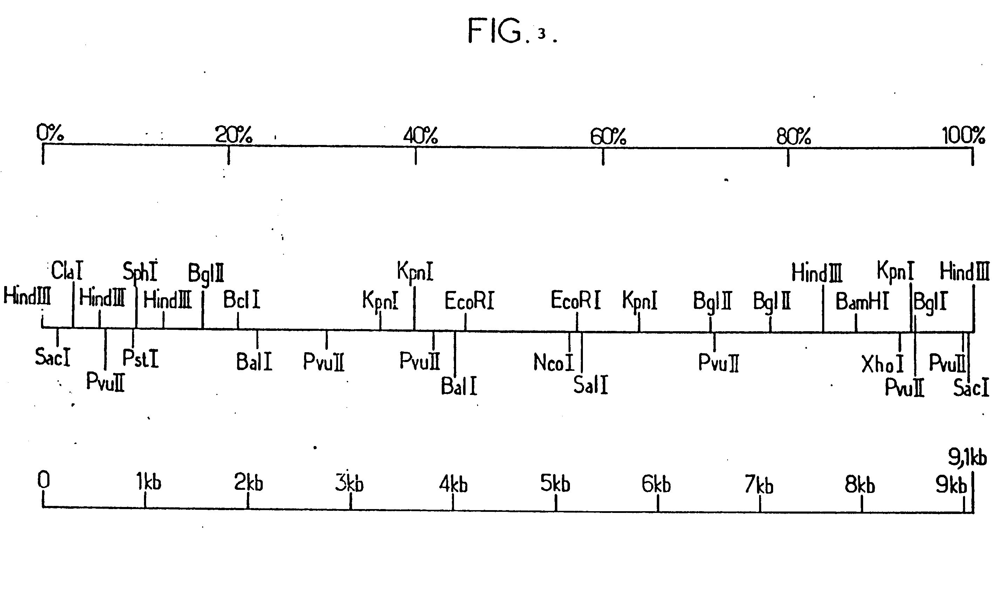

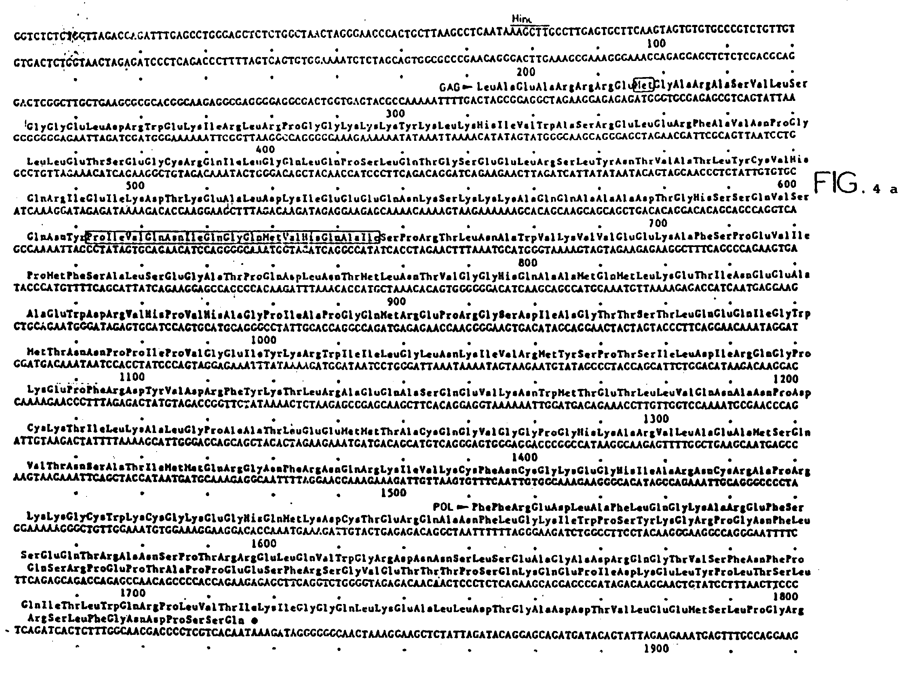

Figures 2 and 3 are restriction maps of such a cDNA comprising a total of 9.1 to 9.2 kb. The polypeptides encoded by cDNA fragments are located in the region extending between site KpnI (position 6100) and site BgIII (position 9150) of the restriction map of Figure 2. The presence of a characteristic site in an envelope antigen of the LAV virus or the like in any polypeptide expressed (in a suitable host cell transformed beforehand by a corresponding fragment or by a vector containing said fragment) can be detected by any suitable immunochemical means.

The invention relates more particularly to polypeptides encoded by cDNA fragments defined hereafter. It also relates to the nucleic acid fragments themselves, including a cDNA variant corresponding to a whole LAV retroviral genome, characterized by a series of restriction sites in the order hereafter (from the 5' end to the 3' end).

The coordinates of the successive sites of the whole LAV genome (see also restriction map of $\lambda$J19 in fig.

2) are indicated hereafter too, with respect to the Hind III site (selected as of coordinate 1) which is located in the R region. The coordinates are estimated with an accuracy of ± 200 bp :

| | |
|---|---|
| Hind III | 0 |
| Sac I | 50 |
| Hind III | 520 |
| Pst I | 800 |
| Hind III | 1 100 |
| Bgl II | 1 500 |
| Kpn I | 3 500 |
| Kpn I | 3 900 |
| Eco RI | 4 100 |
| Eco RI | 5 300 |
| Sal I | 5 500 |
| Kpn I | 6 100 |
| Bgl II | 6 500 |
| Bgl II | 7 600 |
| Hind III | 7 850 |
| Bam HI | 8 150 |
| Xho I | 8 600 |
| Kpn I | 8 700 |
| Bgl II | 8 750 |
| Bgl II | 9 150 |
| Sac I | 9 200 |
| Hind III | 9 250 |

Another DNA variant according to this invention optionally contains an additional Hind III approximately at the 5 550 coordinate.

Reference is further made to fig. 1 which shows a more detailed restriction map of said whole-DNA (λJ19).

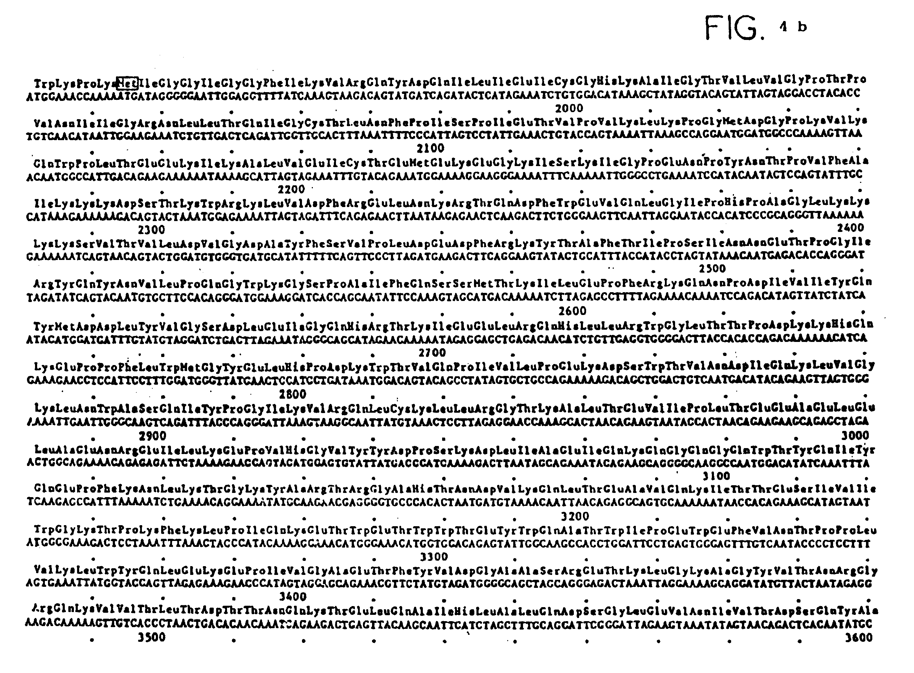

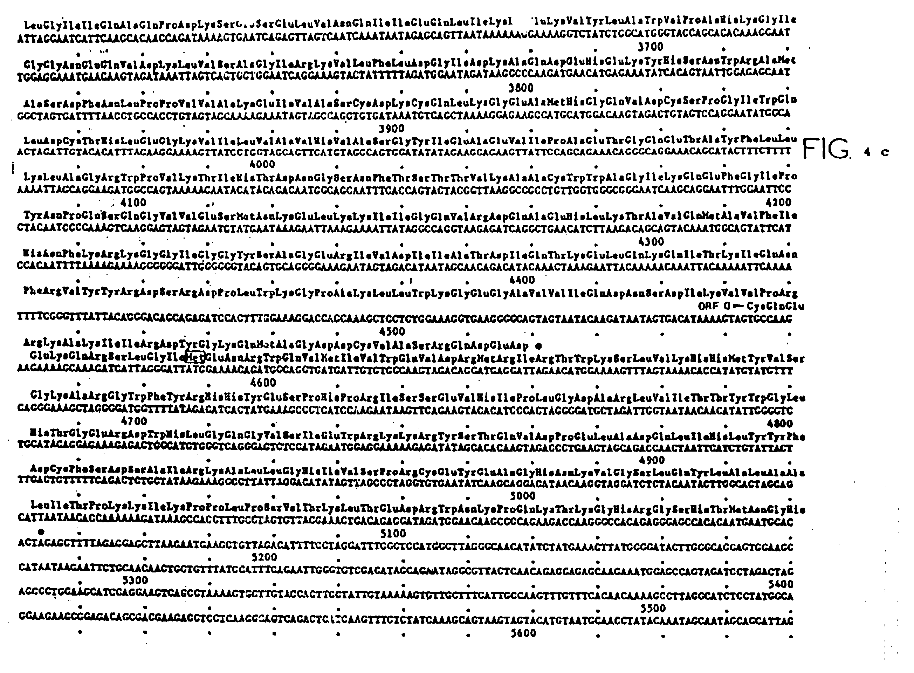

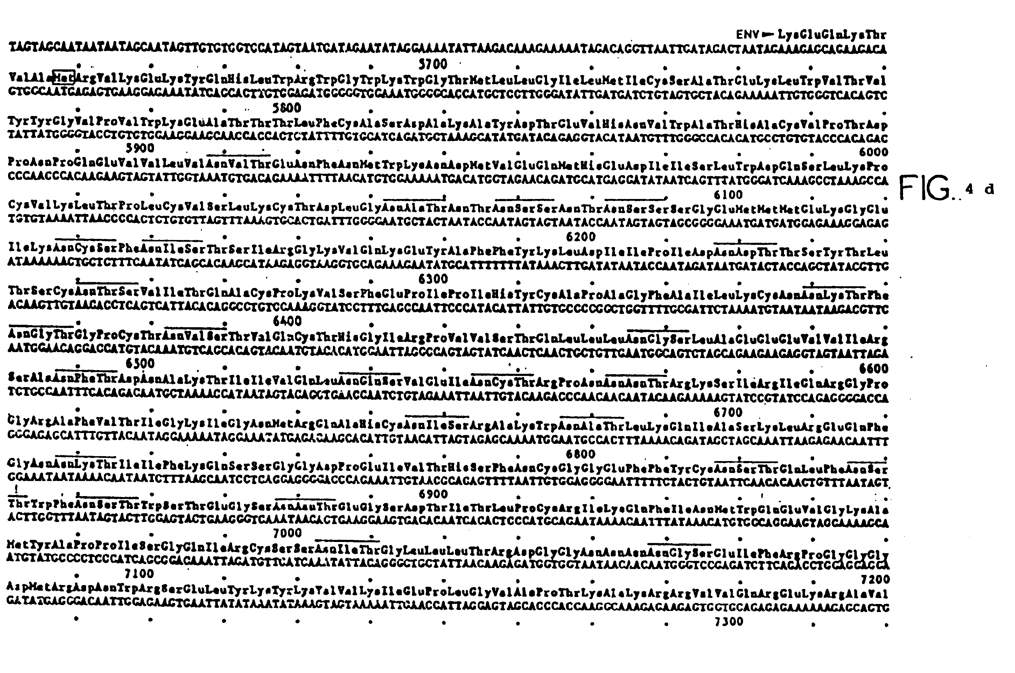

An even more detailed nucleotide sequence of a preferred DNA according to the invention is shown in figs. 4a-4e hereafter.

The invention further relates to other preferred DNA fragments and polypeptide sequences (glycosylated or not glycosylated) which will be referred to hereafter.

## SEQUENCING OF LAV

The sequencing and determination of sites of particular interest were carried out on a phage recombinant corresponding to λJ19 disclosed in the abovesaid EP0178978. A method for preparing it is disclosed in that application.

The whole recombinant phage DNA of clone λJ19 (disclosed in the earlier application) was sonicated according to the protocol of DEININGER (1983), Analytical Biochem. 129, 216. The DNA was repaired by a Klenow reaction for 12 hours at 16°C. The DNA was electrophoresed through 0.8 % agarose gel and DNA in the size range of 300-600 bp was cut out and electroeluted and precipitated. Resuspended DNA (in 10 mM Tris, pH 8 ; 0,1 mM EDTA) was ligated into M13mp8 RF DNA (cut by the restriction enzyme Smal and subsequently alkaline phosphated), using T4 DNA- and RNA-ligases (Maniatis T et al (1982) - Molecular cloning - Cold Spring Harbor Laboratory). An E. coli strain designated as TG1 was used for further study. This strain has the following genotype :

Δlac pro, supE, thi.F'traD36, proAB, lacIq, ZΔM15,r⁻

This E. coli TGI strain has the peculiarity of enabling recombinants to be recognized easily. The blue colour of the cells transfected with plasmids which did not recombine with a fragment of LAV DNA is not modified. To the contrary, cells transfected by a recombinant plasmid containing a LAV DNA fragment yield white colonies. The technique which was used is disclosed in Gene (1983), 26, 101.

This strain was transformed with the ligation mix using the Hanahan method (Hanahan D (1983) J. Mol. Biol. 166, 557). Cells were plated out on a tryptone-agarose plate with IPTG and X-gal in soft agarose. White plaques were either picked and screened or screened directly in situ using nitrocellulose filters. Their DNAs were hybridized with nick-translated DNA inserts of pUC18 Hind III subclones of λJ19. This permitted the isolation of the plasmids or subclones of λ which are identified in the table hereafter. In relation to this table it should also be noted that the designation of each plasmid is followed by the deposition number of a cell culture of E. coli TGI containing the corresponding plasmid at the "Collection Nationale des Cultures de Micro-organismes" (C.N.C.M.) of the Pasteur Institute in Paris, France. A non-transformed TGI cell line was also deposited at the C.N.C.M. under Nr. I-364. All these deposits took place on November 15, 1984. The sizes of the corresponding inserts derived from the LAV genome have also been indicated.

TABLE

Essential features of the recombinant plasmids

- pJ19 - 1 plasmid        (I-365)            0.5 kb

Hind III - Sac I - Hind III

- pJ19 - 17 plasmid       (I-367)            0.6 kb

Hind III - Pst 1 - Hind III

- pJ19 - 6 plasmid        (I-366)            1.5 kb

Hind III (5')
Bam HI
Xho I
Kpn I
Bgl II
Sac I (3')
Hind III

- pJ19-13 plasmid         (I-368)            6.7 kb

Hind III (5')
Bgl II
Kpn I
Kpn I
Eco RI
Eco RI
Sal I
Kpn I
Bgl II
Bgl II
Hind III (3')

Positively hybridizing M13 phage plates were grown up for 5 hours and the single-stranded DNAs were extracted.

M13mp8 subclones of λJ19 DNAs were sequenced according to the dideoxy method and technology devised by Sanger et al (Sanger et al (1977), Proc. Natl. Acad. Sci. USA, 74 , 5463 and M13 cloning and sequencing handbook, AMERSHAM (1983). The 17-mer oligonucleotide primer α-$^{35}$SdATP (400Ci/mmol, AMERSHAM), and 0.5X-5X buffer gradient gels (Biggen M.D. et al (1983, Proc. Natl. Acad. Sci. USA, 50, 3963) were used. Gels were read and put into the computer under the programs of Staden (Staden R. (1982), Nucl. Acids Res. 10, 4731). All the appropriate references and methods can be found in the AMERSHAM M13 cloning and sequencing handbook.

The complete DNA sequence of λJ19 (also designated as LAV-Ia) is shown in figs. 4 to 4e.

The sequence was reconstructed from the sequence of phage λJ19 insert. The numbering starts at the cap site which was located experimentally (see hereafter). Important genetic elements, major open reading frames and their predicted products, are indicated together with the HindIII cloning sites. The potential glycosylation sites in the env gene are overlined. The NH$_2$-terminal sequence of p25$^{gag}$ determined by protein microsequencing is boxed.

Each nucleotide was sequenced on average 5.3 times : 85 % of the sequence was determined on both strands and the remainder sequenced at least twice from independent clones. The base composition is T, 22.2 % ; C, 17.8 % ; A, 35.8 % ; G, 24.2 % ; G + C, 42 %. The dinucleotide GC is greatly under represented (0.9 %) as is common amongst eukaryotic sequences (Bird 1980).

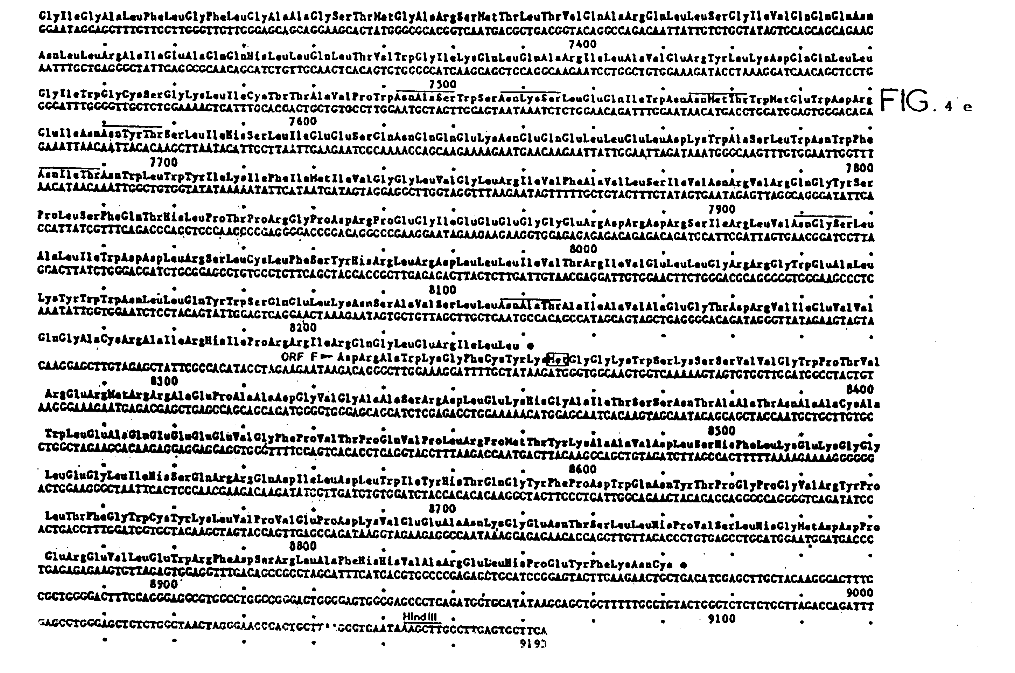

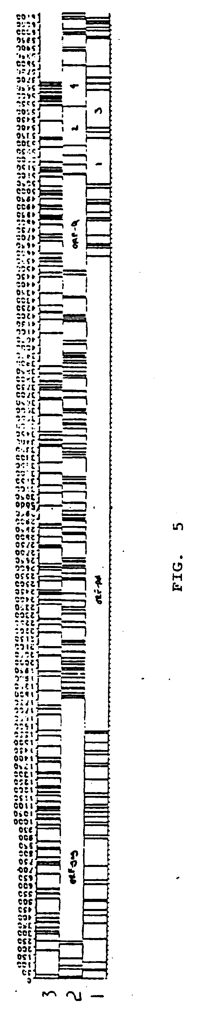

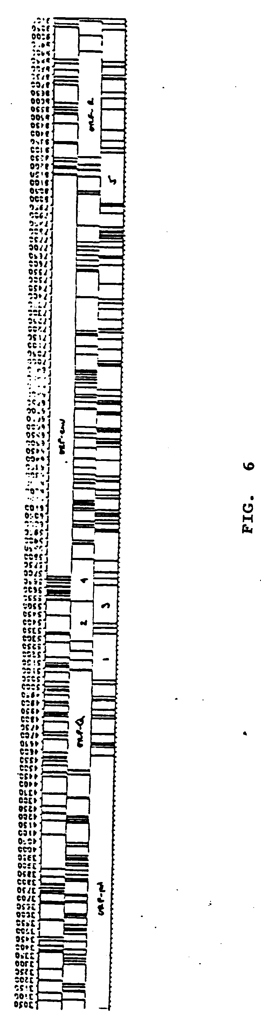

Figs. 5 and 6 provide a diagrammatized representation of the lengths of the successive open reading frames corresponding to the successive reading phases (also referred to by numbers "1", "2" and "3" appearing in the left handside part of fig. 5). The relative positions of these open reading frames (ORF) with respect to the nucleotidic structure of the LAV genome is referred to by the scale of numbers representative of the respective positions of the corresponding nucleotides in the DNA sequence. The vertical bars correspond to the positions of the corresponding stop codons.

The following genes and DNA fragments can be distinguished on the different reading frames shown. Reference is then also made to the proteins or glycoproteins encoded by said genes and fragments. The env gene and other genes are also referred to in the table hereafter.

## The envelope gene (or ORF-env)

env : The env open reading frame has a possible initiator methionine codon very near the beginning (8th triplet). If so the molecular weight of the presumed env precursor protein (861 aminoacids, MWcalc = 97376) is consistent with the size of the LAV glycoprotein (110 kd and 90 kd after glycosidase treatment). There are 32 potential N-glycosylation sites (Asn-X-Ser/Thr) which are overlined in Fig. 4d and 4e. An interesting feature of env is the very high number of Trp residues at both ends of the protein.

The DNA sequence thought to code for envelope proteins is thought to extend from nucleotide position 5746 (starting with 5' AAA GAG GAG A....3') up to nucleotidic position 8208 (ending by .....A ACT AAA GAA 3'). Polypeptide structures of sequences of the envelope protein correspond to those read according to the "phase 3" reading phase.

The start of env transcription is thought to be at the level of the ATG codon at position 5767-5769.

There are three hydrophobic regions, characteristic of the retroviral envelope proteins (Seiki et al., 1983) corresponding to a signal peptide (encoded by nucleotides 5815-5850 bp), a second region (7315-7350 bp) and a transmembrane segment (7831-7890 bp). The second hydrophobic region (7315-7350 bp) is preceded by a stretch rich in Arg + Lys. It is possible that this represents a site of proteolytic cleavage, which by analogy with other retroviral proteins, would give an external envelope polypeptide and a membrane associated protein (Seiki et al., 1983, Kiyokawa et al., 1984). A striking feature of the LAV envelope protein sequence is that the segment following the transmembrane segment is of unusual length (150 residues). The env protein shows nohomology to any sequence in protein data banks. The small aminoacid motif common to the transmembrane proteins of all leukemogenic retroviruses (Cianciolo et al., 1984) is not present in lav env.

The invention describes more particularly the DNA sequence extending from nucleotide 5767, up to nucleotide 8208 or even 8350 deemed to encode gp 110 (envelope glycoprotein of the LAV viruses which has a molecular weight of about 110.000 daltons) beginning at about nucleotide as well as the polypeptidic backbone of the glycoprotein sequence which corresponds to that having an approximate molecular weight of 90.000 daltons and which is obtained by glycosidase treatment of gp110.

The invention further relates to the purified polypeptides which have the aminoacid structure (or polypeptidic backbone) of gp110 and gp90 respectively, which correspond to the direct translation of the DNA sequences and fragments which have been defined more specifically hereabove (figs 4d and 4e).

The invention further relates to polypeptides containing neutralizing epitopes.

The locations of neutralizing epitopes are further apparent in fig. 4d. Reference is more particularly made to the overlined groups of three letters included in the aminoacid sequences of the envelope proteins (reading phase 3) which can be designated generally by the formula Asn-X-Ser or Asn-X-Thr, wherein X is any other possible aminoacid. Thus the initial protein product or polypeptide backbone of the env glycoprotein has a molecular weight in excess of 91,000. These groups are deemed to generally carry glycosylated groups. These Asn-X-Ser and Asn-X-Thr groups with attached glycosylated groups form together hydrophylic regions of the protein and are deemed to be located at the periphery of and to be exposed outwardly with respect to the normal conformation of the proteins. Consequently they are considered as being epitopes which can efficiently be brought into play in vaccine compositions.

The invention thus concerns more particularly peptide sequences included in the env-proteins and excizable therefrom for having the same aminoacid structure), having sizes not exceeding 200 aminoacids.

Preferred peptides of this invention (referred to hereafter as a, b, c, d, e, f) are deemed to correspond to those encoded by the nucleotide sequences which extend respectively between the following positions :

```
a)  from about   6171 to about 6276
b)    "     "    6337  "    "    6387
c)    "     "    6466  "    "    6516
d)    "     "    6562  "    "    6696
e)    "     "    6937  "    "    7005
f)    "     "    7612  "    "    7746
```

Other hydrophilic peptides in the env open reading frame are identified hereafter. They are defined starting from aminoacid (1 = lysine) encoded by the AAA at position 5746-5748 in the LAV DNA sequence (figs 4d and 4e) and then further numbered in accordance with their order with respect to the end sequence. The first and second numbers in relation to each peptide refer to their respective N-terminal and C-terminal aminoacids.

These hydrophilic peptides are :

aminoacids 8-23 inclusive, i.e. Met-Arg-Val-Lys-Glu-Lys-Tyr-Gln-His-Leu-Trp-Arg-Trp-Gly-Trp-Lys-

aminoacids 63-78 inclusive, i.e. Ser-Asp-Ala-Lys-Ala-Tyr-Asp-Thr-Glu-Val-His-Asn-Val-Trp-Ala-Thr-

aminoacids 82-90 inclusive, i.e. Val-pro-Thr-Asp-Pro-Asn-Pro-Gln-Glu-

aminoacids 97-123 inclusive, i.e. Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-

aminocids 127-183 inclusive, i.e. Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

aminoacids 191-201 inclusive, i.e. Leu-Asp-Ile-Ile-Pro-Ile-Asp-Asn-Asp-Thr-Thr-

aminocids 239-294 inclusive, i.e. Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

aminocids 300-327 inclusive, i.e. Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

aminoacids 334-381 inclusive, i.e. Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

aminoacids 397-424 inclusive, i.e. Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

aminoacids 466-500 inclusive, i.e. Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

aminoacids 510-523 inclusive, i.e. Pro-Thr-Lys-Ala-Lys-Arg-Arg-Val-Val-Gln-Arg-Glu-Lys-Arg-

aminoacids 551-577 inclusive, i.e. Val-Gln-Ala-Arg-Gln-Leu-Leu-Ser-Gly-Ile-Val-Gln-Gln-Gln-Asn-Asn-Leu-Leu-Arg-Ala-Ile-Glu-Ala-Gln-Gln-His-Leu-

aminoacids 594-603 inclusive, i.e. Ala-Val-Glu-Arg-Tyr-Leu-Lys-Asp-Gln-Gln-

aminoacids 621-630 inclusive, i.e. Pro-Trp-Asn-Ala-Ser-Trp-Ser-Asn-Lys-Ser-

aminoacids 657-679 inclusive, i.e. Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-

aminoacids 719-758 inclusive, i.e. Arg-Val-Arg-Gln-Gly-Tyr-Ser-Pro-Leu-Ser-Phe-Gln-Thr-His-Leu-Pro-Thr-Pro-Arg-Gly-Pro-Asp-Arg-Pro-Glu-Gly-Ile-Glu-Glu-Glu-Gly-Gly-Glu-Arg-Asp-Arg-Asp-Arg-Ser-Ile-

aminoacids 780-803 inclusive, i.e. Tyr-His-Arg-Leu-Arg-Asp-Leu-Leu-Leu-Ile-Val-Thr-Arg-Ile-Val-Glu-Leu-Leu-Gly-Arg-Arg-Gly-Trp-Glu-

| orf | 1st triplet | Met | stop | No amino acids | MWcalc |
|---|---|---|---|---|---|
| gag | 312 | 336 | 1836 | 500 | 55841 |
| pol | 1631 | 1934 | 4640 | (1003) | (113629) |
| orf Q | 4554 | 4587 | 5163 | 192 | 22487 |
| env | 5746 | 5767 | 8350 | 861 | 97376 |
| orf F | 8324 | 8354 | 8972 | 206 | 23316 |

Table 1 : Location and sizes of viral open reading frames.

The invention describes more particularly all the DNA fragments which have been more specifically referred to hereabove and which correspond to open reading frames. It will be understood that the man skilled in the art will be able to obtain them all, for instance by cleaving an entire DNA corresponding to the complete genome of a LAV species, such as by cleavage by a partial or complete digestion thereof with a suitable restriction enzyme and by the subsequent recovery of the relevant fragments. The different DNAs disclosed above can be resorted to also as a source of suitable fragments. The techniques disclosed hereafter for the isolation of the fragments which were then included in the plasmids referred to hereabove and which were then used for the DNA sequencing can be used.

Of course other methods can be used. Some of them have been examplified in EP-A-178978. Reference is for instance made to the following methods:

a) DNA can be transfected into mammalian cells with appropriate selection markers by a variety of techniques, calcium phosphate precipitation, polyethylene glycol, protoplast-fusion, etc..

b) DNA fragments corresponding to genes can be cloned into expression vectors for E. coli , yeast- or mammalian cells and the resultant proteins purified.

c) The proviral DNA can be "shot-gunned" (fragmented) into procaryotic expression vectors to generate fusion polypeptides. Recombinant producing antigenically competent fusion proteins can be identified by simply screening the recombinants with antibodies against LAV antigens .

The invention further refers more specifically to DNA recombinants, particularly modified vectors, including any of the preceding DNA sequences and adapted to transform corresponding microorganisms or cells, particularly eucaryotic cells such as yeasts, for instance Saccharomyces cerevisiae, or higher eucaryotic cells, particularly cells of mammals, and to permit expression of said DNA sequences in the corresponding microorganisms or cells.

More particularly the invention relates to such modified DNA recombinant vectors modified by the abovesaid DNA sequences and which are capable of transforming higher eucaryotic cells particularly mammalian cells. Preferably any of the abovesaid sequences are placed under the direct control of a promoter contained in said vectors and which is recognized by the polymerases of said cells, such that the first nucleotide codons expressed correspond to the first triplets of the above-defined DNA-sequences. Accordingly, this invention also relates to the corresponding DNA fragments which can be obtained from LAV genomes or corresponding cDNAs by any appropriate method. For instance such a method comprises cleaving said LAV genomes or cDNAs by restriction enzymes preferably at the level of restriction sites surrounding said fragments and close to the opposite extremities respectively thereof, recovering and identifying the fragments sought according to sizes, if need be, checking their restriction maps or nucleotide sequences (or by reaction with monoclonal antibodies specifically directed against epitopes carried by the polypeptides encoded by said DNA fragments), and further, if need be, trimming the extremities of the fragment, for instance by an exonucleolytic enzyme such as Bal31, for the purpose of controlling the desired nucleotide sequences of the extremities of said DNA fragments or, conversely, repairing said extremities with Klenow enzyme and possibly ligating the latter to synthetic polynucleotide fragments designed to permit the reconstitution of the nucleotide extremities of said fragments. Those fragments may then be inserted in any of said vectors for causing the expression of the corresponding polypeptide by the cell transformed therewith. The corresponding polypeptide can then be recovered from the transformed cells, if need be after lysis thereof, and purified, by methods such as electrophoresis. Needless to say that all conventionnal methods for performing these operations can be resorted to.

The invention also relates more specifically to cloned probes which can be made starting from any DNA fragment according to this invention, thus to recombinant DNAs containing such fragments, particularly any plasmids amplifiable in procaryotic or eucaryotic cells and carrying said fragments.

Using the cloned DNA fragments as a molecular hybridization probe - either by labelling with radionucleotides or with fluorescent reagents - LAV virion RNA may be detected directly in the blood, body fluids and blood products (e.g. of the antihaemophylic factors such as Factor VIII concentrates) and vaccines, i.e. hepatitis B vaccine. It has already been shown that whole virus can be detected in culture supernatants of LAV producing cells. A suitable method for achieving that detection comprises immobilizing virus onto a support, e.g. nitrocellulose filters, etc., disrupting the virion and hybridizing with labelled (radiolabelled or "cold" fluorescent- or enzyme-labelled) probes. Such an approach has already been developed for Hepatitis B virus in peripheral blood (according to SCOTTO J. et al. Hepatology (1983), 3, 379-384).

Probes according to the invention can also be used for rapid screening of genomic DNA derived from the tissue of patients with LAV related symptoms, to see if the proviral DNA or RNA is present in host tissue and other tissues.

A method which can be used for such screening comprises the following steps : extraction of DNA from tissue, restriction enzyme cleavage of said DNA, electrophoresis of the fragments and Southern blotting of genomic DNA from tissues, subsequent hybridization with labelled cloned LAV proviral DNA. Hybridization in situ can also be used.

Lymphatic fluids and tissues and other non-lymphatic tissues of humans, primates and other mammalian species can also be screened to see if other evolutionary related retrovirus exists. The methods referred to hereabove can be used, although hybridization and washings would be done under non stringent conditions.

The DNAs or DNA fragments according to the invention can be used also for achieving the expression of LAV viral antigens for diagnostic purposes.

The invention relates generally to the polypeptides themselves, whether synthesized chemically isolated from viral preparation or expressed by the different DNAs of the invention, particularly by the ORFs or fragments thereof, in appropriate hosts, particularly procaryotic or eucaryotic hosts, after transformation thereof with a suitable vector previously modified by the corresponding DNAs.

More generally, the invention also relates to any of the polypeptide fragments (or molecules, particularly glycoproteins having the same polypeptidic backbone as the polypeptides mentioned hereabove) bearing an epitope characteristic of a LAV protein or glycoprotein, which polypeptide or molecule then has N-terminal and C-terminal extremities respectively either free or, independently from each other, covalently bound to aminoacids other than those which are normally associated with them in the larger polypeptides or glycoproteins of the LAV virus, which last mentioned aminoacids are then free or belong to another polypeptidic sequence. Particularly the invention relates to hybrid polypeptides containing any of the epitope-bearing-polypeptides which have been defined more specifically hereabove, recombined with other polypeptide fragments normally foreign to the LAV proteins, having sizes sufficient to provide for an increased immunogenicity of the epitope-bearing-polypeptide yet, said foreign polypeptide fragments either being immunogenically inert or not interfering with the immunogenic properties of the epitope-bearing-polypeptide.

Such hybrid polypeptides, which may contain up to 150, even 250 aminoacids, usually consist of the expression products of a vector which contained ab initio a nucleic acid sequence expressible under the control of a suitable promoter or replicon in a suitable host, which nucleic acid sequence had however beforehand been modified by insertion therein of a DNA sequence encoding said epitope-bearing-polypeptide.

Said epitope-bearing-polypeptides, particularly those whose N-terminal and C-terminal aminoacids are free, are also accessible by chemical synthesis, according to techniques well known in the chemistry of proteins.

The synthesis of peptides in homogeneous solution and in solid phase is well known.

In this respect, recourse may be had to the method of synthesis in homogeneous solution described by Houbenweyl in the work entitled "Methoden der Organischen Chemie" (Methods of Organic Chemistry) edited by E. WUNSCH., vol. 15-I and II, THIEME, Stuttgart 1974.

This method of synthesis consists of successively condensing either the successive aminoacids in twos, in the appropriate order or successive peptide fragments previously available or formed and containing already several aminoacyl residues in the appropriate order respectively. Except for the carboxyl and aminogroups which will be engaged in the formation of the peptide bonds, care must be taken to protect beforehand all other reactive groups borne by these aminoacyl groups or fragments. However, prior to the formation of the peptide bonds, the carboxyl groups are advantageously activated, according to methods well known in the synthesis of peptides. Alternatively, recourse may be had to coupling reactions bringing into play conventional coupling reagents, for instance of the carbodiimide type, such as 1-ethyl-3-(3-dimethyl-aminopropyl)-carbodiimide. When the aminoacid group used carries an additional amine group (e.g. lysine) or another acid function (e.g. glutamic acid), these groups may be protected by carbobenzoxy or t-butyloxycarbonyl groups, as regards the amine groups, or by t-butylester groups, as regards the carboxylic groups. Similar procedures are available for the protection of other reactive groups. For example, SH group (e.g. in cysteine) can be protected by an acetamidomethyl or paramethoxybenzyl group.

In the case of progressive synthesis, aminoacid by aminoacid, the synthesis starts preferably by the condensation of the C-terminal aminoacid with the aminoacid which corresponds to the neighboring aminoacyl group in the desired sequence and so on, step by step, up to the N-terminal aminoacid. Another preferred technique can be relied upon is that described by R.D. Merrifield in "solid phase peptide synthesis" (J. Am. Chem. Soc., 45, 2149-2154).

In accordance with the Merrifield process, the first C-terminal aminoacid of the chain is fixed to a suitable porous polymeric resin, by means of its carboxylic group, the amino group of said aminoacid then being protected, for example by a t-butyloxycarbonyl group.

When the first C-terminal aminoacid is thus fixed to the resin, the protective group of the amine group is removed by washing the resin with an acid, i.e. trifluoroacetic acid, when the protective group of the amine group is a t-butyloxycarbonyl group.

Then the carboxylic group of the second aminoacid which is to provide the second aminoacyl group of the desired peptide sequence, is coupled to the deprotected amine group of the C-terminal aminoacid fixed to the resin. Preferably, the carboxyl group of this second aminoacid has been activated, for example by dicyclohexylcarbodiimide, while its amine group has been protected, for example by a t-butyloxycarbonyl group. The first part of the desired peptide chain, which comprising the first two aminoacids, is thus obtained. As previously, the amine group is then deprotected, and one can further proceed with the fixing of the next aminoacyl group and so forth until the whole peptide sought is obtained.

The protective groups of the different side groups, if any, of the peptide chain so formed can then be removed. The peptide sought can then be detached from the resin, for example, by means of hydrofluoric acid, and finally recovered in pure form from the acid solution according to conventional procedures.

As regards the peptide sequences of smallest size and bearing an epitope or imunogenic determinant, and more particularly those which are readily accessible by chemical synthesis, it may be required, in order to increase their in vivo immunogenic character, to couple or "conjugate" them covalently to a physiologically acceptable and non toxic carrier molecule.

By way of examples of carrier molecules or macromolecular supports which can be used for making the conjugates according to the invention, will be mentioned natural proteins, such as tetanic toxoid, ovalbumin, serum-albumins, hemocyanins, etc.. Synthetic macromolecular carriers, for example polysines or poly(D-L-alanine)-poly(L-lysine)s, can be used too.

Other types of macromolecular carriers which can be used, which generally have molecular weights higher than 20,000, are known from the literature.

The conjugates can be synthesized by known processes, such as described by Frantz and Robertson in "Infect. and Immunity", 33, 193-198 (1981), or by P.E. Kauffman in Applied and Environmental Microbiology, October 1981, Vol. 42, n° 4, 611-614.

For instance, the following coupling agents can be used : glutaric aldehyde, ethyl chloroformate, watersoluble carbodiimides (N-ethyl-N'(3-dimethylaminopropyl) carbodiimide, HCl), diisocyanates, bis-diazobenzidine, di- and trichloro-s-triazines, cyanogen bromides, benzoquinone, as well as coupling agents mentioned in "Scand. J. Immunol., 1978, vol. 8, p. 7-23 (Avrameas, Ternynck, Guesdon).

Any coupling process can be used for bonding one or several reactive groups of the peptide, on the one hand, and one or several reactive groups of the carrier, on the other hand. Again coupling is advantageously achieved between carboxyl and amine groups carried by the peptide and the carrier or vice-versa in the presence of a coupling agent of the type used in protein synthesis, i.e. 1-ethyl-3-(3-dimethylaminopropyl)-carbodiimide, N-hydroxybenzotriazole, etc.. Coupling between amine groups respectively borne by the peptide and the carrier can also be made with glutaraldehyde, for instance, according to the method described by BOQUET, P. et al. (1982) Molec. Immunol., 19, 1441-1549, when the carrier is hemocyanin.

The immunogenicity of epitope-bearing-peptides can also be reinforced, by oligomerisation thereof, for example in the presence of glutaraldehyde or any other suitable coupling agent. In particular, the invention relates to the water soluble immunogenic oligomers thus obtained, comprising particularly from 2 to 10 monomer units.

The glycoproteins, proteins and polypeptides (generally designated hereafter as "antigens" of this invention, whether obtained in a purified state from LAV virus preparations or by recombinant DNA technology, are useful in processes for the detection of the presence of anti-LAV antibodies in biological media, particularly biological fluids such as sera from man or animal, particularly with a view of possibly diagnosing LAS or AIDS.

Particularly the invention relates to an in vitro process of diagnosis making use of an envelope glycoprotein (or of a polypeptide bearing an epitope of this glycoprotein) for the detection of anti-LAV antibodies in the sera of persons who carry them. Other polypeptides - particular those carrying an epitope of a core protein - can be used too.

A preferred embodiment of the process of the invention comprises :
- depositing a predetermined amount of one or several of said antigens in the wells of a titration micoplate :
- introducing increasing dilutions of the biological fluid, i.e. serum to be diagnosed into these wells :
- incubating the microplate :
- washing carefully the microplate with an appropriate buffer :
- adding to the wells specific labelled antibodies directed against blood immunoglobulins and
- detecting the antigen-antibody-complex formed, which is then indicative of the presence of LAV antibodies in the biological fluid.

Advantageously, the labelling of the anti-immunoglobulin antibodies is achieved by an enzyme selected from among those which are capable of hydrolysing a substrate, which substrate undergoes a modification of its radiation-absorption, at least within a predetermined band of wavelength. The detection of the substrate, preferably comparatively with respect to a control, then provides a measurement of the potential risks or of the effective presence of the disease.

Thus preferred methods immuno-enzymatic or also immunofluorescent Antibody, in particular according to the ELISA technique. Antibody titrations may be determinations by immunofluorescence or direct or indirect immunoenzymatic determinations. Quantitative titrations of antibodies in the sera studied can be made.

The invention also relates to the diagnostic kits, themselves, for the in vitro detection of antibodies against the LAV viruses, which kits comprise any of the polypeptides identified herein, and all the biological and chemical reagents, as well as the equipment, necessary for peforming diagnostic assays. Preferred kits comprise all reagents required for carrying out ELISA assays. Thus preferred kits will include, in addition to any of said polypeptides, suitable buffers and anti-human immunoglobulins, which anti-human immunoglobulins are labelled either with an immunofluorescent molecule or an enzyme molecule. In the latter instance preferred kits then also comprise a substrate hydrolysable by the enzyme and providing a signal, particularly modified absorption of a radiation, at least in a determined wavelength, which signal is then indicative of the presence of antibody in the biological fluid to be assayed with said kit.

The invention also relates to vaccine compositions whose active principle is to be constituted by any of the antigens, i.e. the hereabove disclosed polypeptide whole antigens, particularly purified gp110 or immunogenic fragments thereof, fusion polypeptides or oligopeptides in association with a suitable pharmaceutical or physiologically acceptable carrier.

A first type of preferred active principle is the gp110 immunogen.

Other preferred active principles to be considered in that field consist of the peptides containing less than 200 aminoacid units, preferably less than 150, as deducible for the complete genomes of the LAV viruses, and even more preferably those peptides which contain one or more groups selected from Asn-X-Ser and Asn-X-Ser as defined above . Preferred peptides for use in the production of vaccinating principles are peptides (a) to (f) as defined above. By way of example having no limitative character, there may be mentioned that suitable dosages of the vaccine compositions are those which are effective to elicit antibodies in vivo, in the host, particularly a human host. Suitable doses range from 10 to 500 micrograms of polypeptide, protein or glycoprotein per kg, for instance 50 to 100 micrograms per kg.

The different peptides according to this invention can also be used themselves for the production of antibodies, preferably monoclonal antibodies specific for the different peptides respectively. For the production of hybridomas secreting said monoclonal antibodies, conventional production and screening methods are used. These monoclonal antibodies, which themselves are part of the invention then provide very useful tools for the identification and even determination of relative proportions of the different polypeptides or proteins in biological samples, particularly human samples containing LAV or related viruses.

The invention further relates to the hosts (procaryotic or eucaryotic cells) which are transformed by the above mentioned recombinants and which are capable of expressing said DNA fragments.

Finally, the invention also concerns vectors for the transformation of eucaryotic cells of human origin, particularly lymphocytes, the polymerases of which are capable of recognizing the LTRs of LAV. Particularly said vectors are characterized by the presence of a LAV LTR therein, said LTR being then active as a promoter enabling the efficient transcription and translation in a suitable host of a DNA insert coding for a determined protein placed under its control.

Needless to say that the invention extends to all variants of genomes and corresponding DNA fragments (ORFs) having substantially equivalent properties, all of said genomes belonging to retroviruses which can be considered as equivalents of LAV.

It must be understood that the claims which follow are also intended to cover all equivalents of the products (glycoproteins, polypeptides, DNAs, etc..), whereby an equivalent is a product, i.e. a polypeptide which may distinguish from a determined one defined in any of said claims, say through one or several aminoacids, while still having substantially the same immunological or immunogenic properties. A similar rule of equivalency shall apply to the DNAs, it being understood that the rule of equivalency will then be tied to the rule of equivalency pertaining to the polypeptides which they encode.

REFERENCES

Alizon, M., Sonigo, P., Barré-Sinoussi, F., Chermann, J.C., Tiollais, P., Montagnier, L. and Wain-Hobson, S. (1984). Molecular cloning of lymphadenopathy-associated virus. Nature, in press.

Arya, S.K., Gallo, R.C., Hahn, B.H., Shaw, G.M., Popovic, M., Salahuddin, S.Z. and Wong-Staal, F. (1984). Homology of genome of AIDS-associated virus with genomes of human T-cell leukemia lymphoma viruses. Science 225, 927-930.

Barré-Sinoussi, F., Chermann, J.C., Rey, F., Nugeybe, M.T., Chamaret, S.. Gruest, J., Dauguet, C., Axler-Blin, C., Vésinet-Brun F., Rouzioux, C., Rozenbaum, W. and Montagnier, L. (1983). Isolation of a T-lymphotropic retrovirus from a patient at risk of Acquired Immune Deficiency Syndrome (AIDS). Science 220, 868-870.

Biggen, M.D., Gibson, T.J. and Hong, G.F. (1983). Buffer gradient gels and $^{35}$S label as an aid to rapid DNA sequence determination. Proc. Natl. Aca. Sci. USA 80, 3963-3965.

Bird, A.P. (1980). DNA methylation and the frequency of CpG in animal DNA. Nucl. Acids Res. 8, 1799-1504.

Brun-Vézinet, F., Rouzioux, C., Barré-Sinoussi, F., Klatzmann, D., Saimot, A.G., Rozembaum, W., Montagnier, L. and Chermann, J.C. (1984). Detection of IgG antibodies to lymphadenopathy associated virus (LAV) by ELISA, in patients with acquired immuno-deficiency syndrome of lymphadenopathy syndrome. Lancet I, 1253-1256.

Chen, H.R. and Barker, W.C. (1984). Nucleotide sequences of the retroviral long terminal repeats and their adjacent regions. Nucl. Acids Res. 12, 1767-1773.

Chen, I.S.Y., Mc Laughlin, J., Gasson, J.C., Clark, S.C. and Golde, D.W. (1983). Molecular characterization of genome of a novel human T-cell leukaemia virus. Nature 305, 502-505.

Chiu, I.M., Callahan, R., Tronick, S.R., Scholm, J. and Aaronson, S.A. (1984). Major pol gene progenitors in the evolution of oncornaviruses. Science 223, 364-370.

Cianciolo, G.J., Kipnis, R.J. and Snyderman, R. (1984). Similarity between p15E of murine and feline viruses and p21 of HTLV. Nature 311, 515.

Daly, H.M. and Scott, G.L. (1983). Fatal AIDS in a haemophiliae in the U.K. Lancet II, 1190.

Dittmar, K.J. and Moelling, K. (1978). Biochemical properties of p15-associated protease in an avion RNA tumor virus. J. Virol. 28, 106-118.

Donehower, L.A., Huang, A.L. and Hager, G.L. (1981). Regulatory and coding potential of the mouse mammary tumour virus long terminal redundancy. J. Virol. 37, 226-238.

Gottlieb, M.S., Schroff, R., Schanler, H.M., Weisman, J.D., Fan P.T., Wolf R.A., Saxon, A. (1981). Pneumocytis carinii pneumonia and mucosal candidiasis in previously healthy homosexual men : Evidence of a new acquired cellular immuno-deficiency. N. Engl. J.Med. 305, 1426-1431.

Hahn, B.H., Shaw, G.M., Arya, S.U., Popovic, M., Gallo, R.C. and Wong-Stall, F. (1984). Molecular cloning and characterization of the HTLV-III virus associated with AIDS. Nature 312, 166-169.

Harris, J.D., Scott, J.V., Taylor, B., Brahic, M., Stowring, L., Ventura, P., Haase, A.T. and Peluso, R. (1981). Visna virus DNA : discovery of a novel gapped structure. Virology 113, 573-583.

Klyokawa, T., Yoshikura, H., Hattori, S., Secki, M. and Yoshida, M. (1984). Envelope proteins of human T-cell leukemia virus : expression in Escherischia coli and its application to studies of env gene functions. Proc. Natl. Acad. Sci. USA 81, 6202-6206.

Klatzmann, D., Barré-Sinoussi, F., Nugeyre, M.T., Dauguet, C., Vilmer, E., Griscelli, C., Brun-Vézinet, F., Rouzioux, C., Gluckman, J.C., Chermann, J.C. and Montagnier, L. (1984). Selective tropism of lymphadenopathy associated virus (LAV) for helper-inducer T-lymphocytes. Sciences 225, 59-63.

Kozak, M. (1984). Compilation and analysis of sequences upstream from the transcriptional start site in eucaryotic mRNAs. Nucl. Acids Res. 12, 857-872.

Levy, J.A., Hoffman, A.D., Kramer, S.M., Lanois, J.A., Shimabukuro, J.M. and Oskiro, L.S. (1987). Isolation of lymphocytopathic retroviruses from San Francisco patients with AIDS. Science 225, 840-842.

Masur, H., Michelis, M.A., Greene, J.B., Onovato, I., Van de Stowe, R.A., Holzman, R.S., Wormser, G., Brettman, L., Lange, M., Murray, H.W., Cunningham-Rundles, S. (1981). An outbreak of community-acquired pneumocystis carinii pneumonia : Initial manifestation of cellular immune dysfunction. N. Engl. J. Med. 305, 1431-1438.

Misra, T.K., Grandgenett, D.P. and Parsons, J.T. (1982). Avian retrovirus pp32 DNA-binding protein. I. Recognition fo specific sequences on retrovirus DNA terminal repeats. J. Virol. 44, 330-343.

Montagnier, L., et al., (1984). A new human T-lymphotropic retrovirus : characterization and possible role in lymphadenopathy and acquired immune deficiency syndromes. In human T-cell leukemia/lymphoma viruses. R.C. Gallo, M. Essex and L. Gross, eds. (Cold Spring Laboratory, New-York), pp 363-370.

Croszlan, S., Copeland, T.D., Kalyanaraman, V.S., Sarngadharan, M.G., Schultz, A.M. and Gallo, R.C. (1984). Chemical analysis of human T-cell leukemia virus structural proteins. In HTLVS (R.C. Gallo, M.E. Essex and L. Gross, eds) Cold Spring Laboratory, New-York, pp 101-110.

Piot, P., Quinn, T.C., Taelmann, H., Feinsod, F.M. et al., (1984). Acquired immunodeficiency syndrome in a heterosexual population in Zaire. Lancet II, 65-69.

Popovic, M., Sarngadharan, M.G., Read, E. and Gallo, R.C. (1984). Detection, isolation, and continuous production of cytopathic retroviruses (HTLV-III) from patients with AIDS and pre-AIDS. Science 224, 497-500.

Querat, G., Barban, N., Sauze, N., Filippi, P., Vigne, R., Russo, P. and Vitu, C. (1984). Highly lytic and persistent lentiviruses naturally present in sheep with progressive pneumonia are genetically distinct. J. Virol. 52, 672-679.

Raba, M., Limburg, K., Burghagen, M., Katze, J.R., Simsek, M., Heckman, J.E., Rajbhandary, U.L., and Gross, H.J. (1979). Nucleotide sequence fo three isoaccepting lysine tRNAs from rabbit liver and SV40-transformed mouse fibroblasts. Eur. J. Biochem. 97, 305-318.

Rice, N.R., Stephens, R.M., Couez, D., Deschamps, J., Kettmann, R., Burny, A., and Gilden, R.V. (1984). The nucleotide sequence of the env gene and post-env region of bovine leukemia virus. Virology 138, 82-93.

Sagata, N., Yasunaga, T., Ogawa, Y., Tsuzuku-Kawamura, J. and Ikawa, Y. (1984). Bovine leukemia virus : Unique structural features of its long terminal repeats and its evolutionary relationship to human T-cell leukemia virus. Proc. Natl. Acad. Sci. USA 81, 4741-4745.

Sanger, F., Nicklen, S. and Coulsen, A.R. (1977). DNA sequencing with chain terminating inhibitors. Proc. Natl. Acad. Sci. USA 74, 5463-5467.

Schwartz, D.E., Tizard, R. and Gilbert, W. (1983). Nucleotide sequence of Rous sarcoma virus. Cell 32, 853-869.

Schüpbach, J., Popovic, M., Gilden, R.V., Gonda, M.A., Sarngadharan, M.G. and Gallo, R.C. (1984). Serological analysis of a subgroup of human T-lymphotropic retroviruses (HTLV-III) associated with AIDS. Science 224, 503-505.

Seiki, M., Hattori, S., Hirayama, Y. and Yoshida, M. (1983). Human adult T-cell leukemia virus : complete nucleotide sequence of the provirus genome integrated in leukemia cell DNA. Proc. Natl. Acad. Sci. USA 80, 3618-3622.

Shaw, G.M., Hahn, B.H., Arya, S.K., Groopman, J.E., Gallo, R.C. and Wong-Staal, F. (1984). Molecular characterization of human T-cell leukemia (lymphotropic) virus type III in the Acquired Immune Deficiency Syndrome. Science 226, 1165-1171.

Shimotohno, k., and Temin, H.M. (1982). Spontaneous variation and synthesis in the U3 region of the long terminal repeat of avion retroviruses. J. Virol. 41, 1636171.

Shimotohno, K., Golde, D.M., Miwa, M., Sugimura, T. and Chen, I.S.Y. (1984). Nucleotide sequence analysis to the long terminal repeat of human T-cell leukemia virus type II. Proc. Natl. Acad. Sci. USA 81, 1079-1083.

Shinnick, T.M., Lerner, R.A. and Sutcliffe, J.G. (1981). Nucleotide sequence of Moloney murine leukemia virus. Nature 293, 543-548.

Srinivasan, A., Reddy, E.P., Dunn, C.Y. and Aaronson, S.A. (1984). Molecular dissection of transcriptional control elements with the long terminal repeat of retrovirus. Science 223, 286-289.

Staden, R. (1982). Automation of the computer handling of gel reading data produced by the shotgun method of DNA sequencing. Nucl. Acids. Res. 10, 4731-4751.

Temin, H. (1981). Structure, variation and synthesis of retrovirus long terminal repeat. Cell 27, 1-3.

Weinberg, R.A. (1982). Fewer and fewer oncogenes. Cell 30, 3-9.

## Claims

### Claims for the the following Contracting States : BE, CH, DE, FR, GB, IT, LI, LU, NL, SE

1. Purified peptide excizable from a purified envelope glycoprotein of the LAV virus having a molecular weight of the order of 110.000, as measurable by its migration distance in a migration system in comparison with the distances of standard proteins having known molecular weights in the same migration system, said glycoprotein providing upon treatment with endoglycosidases and subsequent removal of the sugar residues, a protein having a molecular weight of about 90.000 daltons as measurable in the same migration system or from a polypeptide which has the same polypeptide backbone structure as said glycoprotein, which peptide has a size not exceeding 200 aminoacids,

2. Purified peptide characterized in that it distinguishes from a purified peptide according to claim 1, while still having substantially the same immunological or immunogenic properties as the corresponding non-modified peptide, such peptides having for example one or several aminoacids different from those of a determined peptide according to claim 1.

3. Purified peptide according to claim 1 or 2, characterised in that it contains at least one neutralizing epitope, such an epitope comprising more particularly an amino acid sequence having the formula Asn-X-Ser or Asn-X-Thr, wherein X is any other possible aminoacid.

4. Purified peptide according to claim 1 or 2 characterised in that it contains at least a neutralizing epitope, such an epitope comprising more particularly an amino acid sequence having the formula Asn-X-Ser or Asn-X-Thr, wherein X is any other possible aminoacid and the epitope is glycosylated or not.

5. Purified peptide according to any of claims 1 to 4 characterised in that it is encoded by the nucleotide sequence which extend respectively between the following positions:
   a/ nucleotides 6171 to 6276 and which has the following aminoacid sequence

```
AsnAlaThrAsnThrAsnSerSerAsnThrAsnSerSerSerGlyGluMetMet
MetGluLysGlyGluIleLysAsnCysSerPheAsnIleSerThrSerIle
```

or
b/ nucleotides 6337 to 6387 and which has the following aminoacid sequence

```
        AsnAspThrThrSerTyrThrLeuThrSerCysAsnThrSerValIleThr
```

c/ nucleotides 6466 to 6516 and which has the following aminoacid sequence

```
    AsnAsnLysThrPheAsnGlyThrGlyProCysThrAsnValSerThrVal
```

or
d/ nucleotides 6562 to 6696 and which has the following aminoacid sequence

```
LeuAsnGlySerLeuAlaGluGluGluValValIleArgSerAlaAsnPheThr
AspAsnAlaLysThrIleIleValGlnLeuAsnGlnSerValGluIleAsnCys
ThrArgProAsnAsnAsnThrArgLys, or
```

e/ nucleotides 6937 to 7005 and which has the following aminoacid sequence

```
AsnSerThrGlnLeuPheAsnSerThrTrpPheAsnSerThrTrpSerThrGlu
GlySerAsnAsnThr, or
```

f/ nucleotides 7612 to 7746 and which has the following aminoacid sequence

```
AsnAlaSerTrpSerAsnLysSerLeuGluGlnIleTrpAsnAsnMetThrTrp
MetGluTrpAspArgGluIleAsnAsnTyrThrSerLeuIleHisSerLeuIle
GluGluSerGlnAsnGlnGlnGluLys
```

6. Purified peptide according to claim 1 or 2, characterised in that it is selected from the following group, the first and last aminoacids corresponding respectively to the N-terminal and C-terminal aminoacids, aminoacids 8-23 inclusive, i.e. Met-Arg-Val-Lys-Glu-Lys-Tyr-Gln-His-Leu-Trp-Arg-Trp-Gly-Trp-Lys-
aminoacids 63-78 inclusive, i.e. Ser-Asp-Ala-Lys-Ala-Tyr-Asp-Thr-Glu-Val-His-Asn-Val-Trp-Ala-Thr-
aminoacids 82-90 inclusive, i.e. Val-Pro-Thr-Asp-Pro-Asn-Pro-Gln-Glu-
aminoacids 97-123 inclusive, i.e. Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-
aminoacids 127-183 inclusive, i.e. Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-
aminoacids 191-201 inclusive, i.e. Leu-Asp-Ile-Ile-Pro-Ile-Asp-Asn-Asp-Thr-Thr-
aminoacids 239-294 inclusive, i.e. Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-
aminoacids 300-327 inclusive, i.e. Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-
aminoacids 334-381 inclusive, i.e. Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-
aminoacids 397-424 inclusive, i.e. Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-
aminoacids 466-500 inclusive, i.e. Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-
aminoacids 510-523 inclusive, i.e. Pro-Thr-Lys-Ala-Lys-Arg-Arg-Val-Val-Gln-Arg-Glu-Lys-Arg-
aminoacids 551-577 inclusive, i.e. Val-Gln-Ala-Arg-Gln-Leu-Leu-Ser-Gly-Ile-Val-Gln-Gln-Gln-Asn-Asn-
Leu-Leu-Arg-Ala-Ile-Glu-Ala-Gln-Gln-His-Leu-
aminoacids 594-603 inclusive, i.e. Ala-Val-Glu-Arg-Tyr-Leu-Lys-Asp-Gln-Gln-
aminoacids 621-630 inclusive, i.e. Pro-Trp-Asn-Ala-Ser-Trp-Ser-Asn-Lys-Ser-
aminoacids 657-679 inclusive, i.e. Leu-Ile-Glu-Glu-Ser-Gln-Asn-Gln-Gln-Glu-Lys-Asn-Glu-Gln-Glu-Leu-
Leu-Glu-Leu-Asp-Lys-Trp-Ala-
aminoacids 719-758 inclusive, i.e. Arg-Val-Arg-Gln-Gly-Tyr-Ser-Pro-Leu-Ser-Phe-Gln-Thr-His-Leu-Pro-
Thr-Pro-Arg-Gly-Pro-Asp-Arg-Pro-Glu-Gly-Ile-Glu-Glu-Glu-Gly-Gly-Glu-Arg-Asp-Arg-Asp-Arg-Ser-Ile-
aminoacids 780-803 inclusive, i.e. Tyr-His-Arg-Leu-Arg-Asp-Leu-Leu-Leu-Ile-Val-Thr-Arg-Ile-Val-Glu-
Leu-Leu-Gly-Arg-Arg-Gly-Trp-Glu-

7. Purified peptide according to claims 3 and 4 characterised in that it is selected from the following group, the first and last aminoacids corresponding respectively to the N-terminal and C-terminal aminoacids,
aminoacids 127-183 inclusive, i.e. Val-Lys-Leu-Thr-Pro-Leu-Cys-Val-Ser-Leu-Lys-Cys-Thr-Asp-Leu-Gly-
Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-
Lys-Asn-Cys-Ser-Phe-Asn-Ile-Ser-Thr-Ser-Ile-Arg-Gly-Lys-Val-Gln-Lys-
aminoacids 197-201 inclusive, i.e. Leu-Asp-Ile-Ile-Pro-Ile-Asp-Asn-Asp-Thr-Thr-
aminoacids 239-294 inclusive, i.e. Lys-Cys-Asn-Asn-Lys-Thr-Phe-Asn-Gly-Thr-Gly-Pro-Cys-Thr-Asn-
Val-Ser-Thr-Val-Gln-Cys-Thr-His-Gly-Ile-Arq-Pro-Val-Val-Ser-Thr-Gln-Leu-Leu-Leu-Asn-Gly-Ser-Leu-
Ala-Glu-Glu-Glu-Val-Val-Ile-Arg-Ser-Ala-Asn-Phe-Thr-Asp-Asn-Ala-Lys-
aminoacids 300-327 inclusive i.e. Leu-Asn-Gln-Ser-Val-Glu-Ile-Asn-Cys-Thr-Arg-Pro-Asn-Asn-Asn-Thr-
Arg-Lys-Ser-Ile-Arg-Ile-Gln-Arg-Gly-Pro-Gly-Arg-
aminoacids 334-381 inclusive, i.e. Lys-Ile-Gly-Asn-Met-Arg-Gln-Ala-His-Cys-Asn-Ile-Ser-Arg-Ala-Lys-
Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-
Lys-Gln-Ser-Ser-Gly-Gly-Asp-Pro-
aminoacids 397-424 inclusive, i.e. Cys-Asn-Ser-Thr-Gln-Leu-Phe-Asn-Ser-Thr-Trp-Phe-Asn-Ser-Thr-
Trp-Ser-Thr-Glu-Gly-Ser-Asn-Asn-Thr-Glu-Gly-Ser-Asp-
aminoacids 466-500 inclusive, i.e. Leu-Thr-Arg-Asp-Gly-Gly-Asn-Asn-Asn-Asn-Gly-Ser-Glu-Ile-Phe-
Arg-Pro-Gly-Gly-Gly-Asp-Met-Arg-Asp-Asn-Trp-Arg-Ser-Glu-Leu-Tyr-Lys-Tyr-Lys-Val-
aminoacids 621-630 inclusive, i.e. Pro-Trp-Asn-Ala-Ser-Trp-Ser-Asn-Lys-Ser-

8. Purified peptide according to anyone of claims 3 or 7, characterised in that it is an epitope-bearing peptide and particularly in that it is an immunogenic peptide.

9. Purified peptide according to claim 8, under an oligomer form, especially a water soluble immunogenic oligomer,

10. Hybrid polypeptide comprising a peptide according to anyone of claims 3, 4, 7 or 8 and having N-terminal and C-terminal extremities, covalently bond to aminoacids other than those which are normally associated with them in the gp110 of LAV, which last mentioned aminoacids are then free or belong to another polypeptidic sequence, particularly a sequence having a size sufficient to provide for an increased immunogenicity

11. Composition of peptides characterised in that it comprises at least 2 peptides according to anyone of claims 1 to 8.

12. Immunogenic composition comprising a purified peptide according to claim 1, 2, 7 or 8, or a purified oligomer according to claim 9, or a hybrid polypeptide according to claim 10, and a suitable pharmaceutical vehicle.

13. DNA sequence characterised in that it codes for a peptide according to anyone of claims 1 to 8.

14. DNA sequence according to claim 13 characterised in that it is inserted in a vector.

15. Process for the chemical synthesis of a peptide or polypeptide according to anyone of claims 1 to 8, characterised by:
- successively condensing either the successive aminoacids in twos, in the appropriate order or successive peptide fragments previously available or formed and containing already several aminoacyl residues in the appropriate order respectively, all the reactive groups other than the carboxyl and aminogroups which will be engaged in the formation of the peptide bond being protected,
- possibly activating the carboxylgroups prior to the formation of the peptide bond,
- recovering the peptide or polypeptide formed.

16. Process for the preparation of a peptide according to anyone of claims 1 to 8, characterised by:
- the transformation of procaryotic or eucaryotic host with a suitable vector previously modified by the corresponding DNA according to claim 13 in conditions enabling the production of the desired peptide,
- recovering and purifying the peptide thus obtained for instance by electrophoresis.

17. Process for the in vitro diagnosis of the presence of anti-LAV antibodies in biological media characterised in that it comprises contacting said biological fluid with the product of any of claims 1 to 8 under conditions suitable for enabling a complex between said antibodies and said product to be formed and detecting said complex as indicative of the presence of said antibodies in said biological fluid.

18. Kit for the in vitro diagnosis of the presence of antibodies directed against LAV virus in fluids which kit comprises:
- a peptide according to anyone of anyone claims 1 to 8 or a composition according to claim 11,
- biological and chemical reagents necessary for performing the diagnostic assay such as suitable buffers, anti-human immunoglobulins previously by labelled with an fluorescent molecule or an enzyme,
- when the anti-human immunoglobulins are labelled with an enzyme, a substrate hydrolysable by the enzyme and providing a signal indicative of the presence of antibody in the biological fluid.

19. Process for the production of monoclonal antibodies against a purified peptide according to anyone of claims 1 to 9, comprising the step of :
- fusing myeloma cells of an animal previously immunised with this purified peptide, with splenocytes, to obtain hybridomas ;
- selecting the hybridomas producing antibodies having specificity for the purified peptide ;
- recovering the antibodies against the purified peptide.

20. Antibody characterized in that it is recognized by the purified peptide according to anyone of claims 1 to 9.

21. Antibody according to claim 20 characterized in that it is a monoclonal antibody.

22. Use of antibodies according to claim 20 or 21, for the identification of the envelope glycoprotein of LAV virus, or even for the determination of relative proportion of this antigen, in a biological sample containing LAV or related viruses.

**Claims for the following Contracting State : AT**

1. Process for the preparation of a purified peptide excizable from a purified envelope glycoprotein of the LAV virus having a molecular weight of the order of 110.000, as measurable by its migration distance in a migration system in comparison with the distances of standard proteins having known molecular weights in the same migration system, said glycoprotein providing upon treatment with endoglycosidases and subsequent removal of the sugar residues, a protein having a molecular weight of about 90.000 daltons as measurable in the same migration system or from a polypeptide which has the same polypeptide backbone structure as said glycoprotein, which peptide has a size not exceeding 200 aminoacids, characterized by
- the transformation of procaryotic or eucaryotic host with a suitable vector previously modified by the corresponding DNA in conditions enabling the production of the desired peptide,
- recovering and purifying the peptide thus obtained for instance by electrophoresis.

2. Process for the preparation of a purified peptide characterized in that the recovered peptide distinguishes from a purified peptide according to claim 1, while still having substantially the same immunological or immunogenic properties as the corresponding non-modified peptide, such peptide having for example one or several aminoacids different from those of a determined peptide according to claim 1.

3. Process for the preparation of a purified peptide according to claim 1 or 2, characterised in that the recovered peptide contains at least one neutralizing epitope, such an epitope comprising more particularly an amino acid sequence having the formula Asn-X-Ser or Asn-X-Thr, wherein X is any other possible aminoacid.

4. Process for the preparation of a purified peptide according to claim 1 or 2 characterised in that the recovered peptide contains at least a neutralizing epitope, such an epitope comprising more particularly an amino acid sequence having the formula Asn-X-Ser or Asn-X-Thr, wherein X is any other possible aminoacid and the epitope is glucosylated or not.

5. Process for the preparation of a purified peptide according to any of claims 1 to 4 characterized in that the recovered peptide is encoded by the nucleotide sequence which extend respectively between the following positions:

a/ nucleotides 6171 to 6276 and which has the following aminoacid sequence

```
AsnAlaThrAsnThrAsnSerSerAsnThrAsnSerSerSerGlyGluMetMet
MetGluLysGlyGluIleLysAsnCysSerPheAsnIleSerThrSerIle
```

or
b/ nucleotides 6337 to 6387 and which has the following aminoacid sequence

```
AsnAspThrThrSerTyrThrLeuThrSerCysAsnThrSerValIleThr
```

c/ nucleotides 6466 to 6516 and which has the following aminoacid sequence

```
AsnAsnLysThrPheAsnGlyThrGlyProCysThrAsnValSerThrVal
```

or
d/ nucleotides 6562 to 6696 and which has the following aminoacid sequence

```
LeuAsnGlySerLeuAlaGluGluGluValValIleArgSerAlaAsnPheThr
AspAsnAlaLysThrIleIleValGlnLeuAsnGlnSerValGluIleAsnCys
ThrArgProAsnAsnAsnThrArgLys, or
```

e/ nucleotides 6937 to 7005 and which has the following aminoacid sequence

```
AsnSerThrGlnLeuPheAsnSerThrTrpPheAsnSerThrTrpSerThrGlu
GlySerAsnAsnThr, or
```

f/ nucleotides 7612 to 7746 and which has the following aminoacid sequence

```
AsnAlaSerTrpSerAsnLysSerLeuGluGlnIleTrpAsnAsnMetThrTrp
MetGluTrpAspArgGluIleAsnAsnTyrThrSerLeuIleHisSerLeuIle
GluGluSerGlnAsnGlnGlnGluLys
```

6. Process for the preparation of a purified peptide according to claim 1 or 2, characterised in that the recovered peptide is selected from the following group, the first and last aminoacids corresponding respectively to the N-terminal and C-terminal aminoacids,
aminoacids 8-23 inclusive, i.e. Met-Arg-Val-Lys-Glu-Lys-Tyr-Gln-His-Leu-Trp-Arg-Trp-Gly-Trp-Lys-
aminoacids 63-78 inclusive, i.e. Ser-Asp-Ala-Lys-Ala-Tyr-Asp-Thr-Glu-Val-His-Asn-Val-Trp-Ala-Thr-
aminoacids 82-90 inclusive, i.e. Val-Pro-Thr-Asp-Pro-Asn-Pro-Gln-Glu-
aminoacids 97-123 inclusive, i.e. Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-
aminoacids 127-183 inclusive, i.e. Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-
aminoacids 191-201 inclusive, i.e. Leu-Asp-Ile-Ile-Pro-Ile-Asp-Asn-Asp-Thr-Thr-
aminoacids 239-294 inclusive, i.e. Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-
aminoacids 300-327 inclusive i.e. Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-
aminoacids 334-381 inclusive, i.e. Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-
aminoacids 397-424 inclusive, i.e. Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-
aminoacids 466-500 inclusive, i.e. Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-
aminoacids 510-523 inclusive, i.e. Pro-Thr-Lys-Ala-Lys-Arg-Arg-Val-Val-Gln-Arg-Glu-Lys-Arg-
aminoacids 551-577 inclusive, i.e. Val-Gln-Ala-Arg-Gln-Leu-Leu-Ser-Gly-Ile-Val-Gln-Gln-Gln-Asn-Asn-Leu-Leu-Arg-Ala-Ile-Glu-Ala-Gln-Gln-His-Leu-
aminoacids 594-603 inclusive, i.e. Ala-Val-Glu-Arg-Tyr-Leu-Lys-Asp-Gln-Gln-
aminoacids 621-630 inclusive, i.e. Pro-Trp-Asn-Ala-Ser-Trp-Ser-Asn-Lys-Ser-
aminoacids 657-679 inclusive, i.e. Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-
aminoacids 719-758 inclusive, i.e. Arg-Val-Arg-Gln-Gly-Tyr-Ser-Pro-Leu-Ser-Phe-Gln-Thr-His-Leu-Pro-Thr-Pro-Arg-Gly-Pro-Asp-Arg-Pro-Glu-Gly-Ile-Glu-Glu-Glu-Gly-Gly-Glu-Arg-Asp-Arg-Asp-Arg-Ser-Ile-
aminoacids 780-803 inclusive, i.e. Tyr-His-Arg-Leu-Arg-Asp-Leu-Leu-Leu-Ile-Val-Thr-Arg-Ile-Val-Glu-Leu-Leu-Gly-Arg-Arg-Gly-Trp-Glu-

7.  Process for the preparation of a purified peptide according to claims 3 and 4 characterised in that the recovered peptide is selected from the following group, the first and last aminoacids corresponding respectively to the N-terminal and C-terminal aminoacids,
aminoacids 127-183 inclusive, i.e. Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-
aminoacids 197-201 inclusive, i.e. Leu-Asp-Ile-Ile-Pro-Ile-Asp-Asn-Asp-Thr-Thr-
aminoacids 239-294 inclusive, i.e. Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arq-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-
aminoacids 300-327 inclusive, i.e. Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-
aminoacids 334-381 inclusive, i.e. Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-
aminoacids 397-424 inclusive, i.e. Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-
aminoacids 466-500 inclusive, i.e. Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-
aminoacids 621-630 inclusive, i.e. Pro-Trp-Asn-Ala-Ser-Trp-Ser-Asn-Lys-Ser-

8.  Process for the preparation of a purified peptide according to anyone of claims 3 or 7, characterised in that the recovered peptide is an epitope-bearing peptide and particularly in that it is an immunogenic peptide.

9.  Process for the preparation of a purified peptide according to claim 8, characterized in that the recovered peptide is then put under an oligomer form, especially a water soluble immunogenic oligomer,

10. Process for the preparation of a hybrid polypeptide comprising a peptide as obtained according to anyone of claims 3, 4, 7 or 8 and having N-terminal and C-terminal extremities, which peptide is covalently bond to aminoacids other than those which are normally associated with them in the gp110 of LAV, which last mentioned aminoacids are then free or belong to another polypeptidic sequence, particularly a sequence having a size sufficient to provide for an increased immunogenicity

11. Process for the preparation of a composition of peptides characterised by mixing at least 2 peptides according to anyone of claims 1 to 8.

12. Process for the preparation of an immunogenic composition characterized by mixing a purified peptide as obtained according to claim 1, 2, 7 or 8, or a purified oligomer according to claim 9, or a hybrid polypeptide according to claim 10, with a suitable pharmaceutical vehicle.

13. Process for the preparation of a DNA sequence characterized by:
    - cleaving the entire DNA corresponding to the complete genome of a LAV species by digestion with a suitable restriction enzyme,
    - recovering the relevant fragment coding for a peptide as obtained according to anyone of claims 1 to 8.

14. Process for the preparation of a DNA sequence according to claim 13 characterised in that the DNA sequence is inserted in a vector.

15. Process for the chemical synthesis of a peptide or polypeptide according to anyone of claims 1 to 8, characterised by:
    - successively condensing either the successive aminoacids in twos, in the appropriate order or successive peptide fragments previously available or formed and containing already several aminoacyl residues in the appropriate order respectively, all the reactive groups other than the carboxyl and aminogroups which will be engaged in the formation of the peptide bond being protected,
    - possibly activating the carboxylgroups prior to the formation of the peptide bond,
    - recovering the peptide or polypeptide formed.

16. Process for the *in vitro* diagnosis of the presence of anti-LAV antibodies in biological media characterised in that it comprises contacting said biological fluid with the product of any of claims 1 to 8 under conditions suitable for enabling a complex between said antibodies and said product to be formed and detecting said complex as indicative of the presence of said antibodies in said biological fluid.

17. Process for the production of monoclonal antibodies against a purified peptide as obtained according to anyone of claims 1 to 9, comprising the step of:
    - fusing myeloma cells of an animal previously immunised with this purified peptide, with splenocytes, to obtain hybridomas;
    - selecting the hybridomas producing antibodies having specificity for the purified peptide;
    - recovering the antibodies agaisnt the purified peptide.

18. Process for the production of antibodies characterized by immunising an animal with a purified peptide as obtained according to claims 1 to 9, recovering the antibodies recognized by the purified peptide according to anyone of claims 1 to 9.

19. Use of antibodies according to claim 17 or 18, for the identification of the envelope glycoprotein of LAV virus, or even for the determination of relative proportion of this antigen, in a biological sample containing LAV or related viruses.

**Patentansprüche**

**Patentansprüche für folgende Vertragsstaaten : BE, CH, DE, FR, GB, IT, LI, LU, NL, SE**

1. Gereinigtes Peptid, herausschneidbar aus einem gereinigten Hüllglycoprotein des LAV-Virus mit einem Molekulargewicht im Bereich von 110 000, meßbar durch seinen Wanderungsabstand in einem Wanderungssystem im Vergleich mit den Abständen von Standardproteinen mit bekannten Molekulargewichten im gleichen Wanderungssystem, wobei das Glycoprotein nach Behandlung mit Endoglycosidasen und nachfolgender Entfernung der Zuckerreste ein Protein mit einem Molekulargewicht von etwa 90 000 Dalton liefert, meßbar im gleichen Wanderungssystem, oder aus einem Polypeptid, das das gleiche Polypeptidgrundgerüst wie obiges Glycoprotein hat, wobei das Peptid eine Größe aufweist, die 200 Aminosäuren nicht überschreitet.

2. Gereinigtes Peptid, dadurch gekennzeichnet, daß es sich von einem gereinigten Peptid nach Anspruch 1 unterscheidet, wobei es im wesentlichen die gleichen immunologischen oder immunogenen Eigenschaften, wie das entsprechende nicht-modifizierte Peptid aufweist, wobei solche Peptide beispielsweise eine oder mehrere Aminosäuren aufweisen, die von denen eines bestimmten Peptids nach Anspruch 1 verschieden sind.

3. Gereinigtes Peptid nach Anspruch 1 oder 2, dadurch gekennzeichnet, daß es mindestens ein neutralisierendes Epitop enthält, wobei ein solches Epitop insbesondere eine Aminosäuresequenz mit der Formel Asn-X-Ser oder Asn-X-Thr umfaßt, wobei X jede andere mögliche Aminosäure ist.

4. Gereinigtes Peptid nach Anspruch 1 oder 2, dadurch gekennzeichnet, daß es mindestens ein neutralisierendes Epitop enthält, wobei ein solches Epitop insbesondere eine Aminosäuresequenz mit der Formel Asn-X-Ser oder Asn-X-Thr umfaßt, wobei X jede andere mögliche Aminosäure ist und das Epitop glycosyliert ist oder nicht.

5. Gereinigtes Peptid nach einem der Ansprüche 1 bis 4, dadurch gekennzeichnet, daß es von der Nucleotidsequenz codiert wird, die sich jeweils zwischen den folgenden Positionen erstreckt:
   a) Nucleotide 6171 bis 6276 mit der folgenden Aminosäuresequenz

**AsnAlaThrAsnThrAsnSerSerAsnThrAsnSerSerSerGlyGluMetMet
MetGluLysGlyGluIleLysAsnCysSerPheAsnIleSerThrSerIle**

oder
   b) Nucleotide 6337 bis 6387 mit der folgenden Aminosäuresequenz

**AsnAspThrThrSerTyrThrLeuThrSerCysAsnThrSerValIleThr**

   c) Nucleotide 6466 bis 6516 mit der folgenden Aminosäuresequenz

**AsnAsnLysThrPheAsnGlyThrGlyProCysThrAsnValSerThrVal**

oder
   d) Nucleotide 6562 bis 6696 mit der folgenden Aminosäuresequenz

**LeuAsnGlySerLeuAlaGluGluGluValValIleArgSerAlaAsnPheThr
AspAsnAlaLysThrIleIleValGlnLeuAsnGlnSerValGluIleAsnCys
ThrArgProAsnAsnAsnThrArgLys,**

oder
   e) Nucleotide 6937 bis 7005 mit der folgenden Aminosäuresequenz

**AsnSerThrGlnLeuPheAsnSerThrTrpPheAsnSerThrTrpSerThrGlu
GlySerAsnAsnThr,**

oder
   f) Nucleotide 7612 bis 7746 mit der folgenden Aminosäuresequenz:

AsnAlaSerTrpSerAsnLysSerLeuGluGlnIleTrpAsnAsnMetThrTrp
MetGluTrpAspArgGluIleAsnAsnTyrThrSerLeuIleHisSerLeuIle
GluGluSerGlnAsnGlnGlnGluLys

6. Gereinigtes Peptid nach Anspruch 1 oder 2, dadurch gekennzeichnet, daß es aus der folgenden Gruppe ausgewählt ist, wobei die erste und letzte Aminosäure jeweils der N-terminalen bzw. C-terminalen Aminosäure entsprechen, Aminosäuren 8-23 einschließlich, nämlich

Met-Arg-Val-Lys-Glu-Lys-Tyr-Gln-His-Leu-Trp-Arg-Trp-Gly-Trp-Lys-

Aminosäuren 63-78 einschließlich, nämlich

Ser-Asp-Ala-Lys-Ala-Tyr-Asp-Thr-Glu-Val-His-Asn-Val-Trp-Ala-Thr-

Aminosäuren 82-90 einschließlich, nämlich

Val-Pro-Thr-Asp-Pro-Asn-Pro-Gln-Glu-

Aminosäuren 97-123 einschließlich, nämlich

Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-

Aminosäuren 127-183 einschließlich, nämlich

Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

Aminosäuren 191-201 einschließlich, nämlich

Leu-Asp-Ile-

Ile-Pro-Ile-Asp-Asn-Asp-Thr-Thr-

Aminosäuren 239-294 einschließlich, nämlich

Lys-Cys-Asn-
Asn-Lys-Thr-Phe-Asn-Gly-Thr-Gly-Pro-Cys-Thr-Asn-
Val-Ser-Thr-Val-Gln-Cys-Thr-His-Gly-Ile-Arg-Pro-
Val-Val-Ser-Thr-Gln-Leu-Leu-Leu-Asn-Gly-Ser-Leu-
Ala-Glu-Glu-Glu-Val-Val-Ile-Arg-Ser-Ala-Asn-Phe-
Thr-Asp-Asn-Ala-Lys-

Aminosäuren 300-327 einschließlich, nämlich

Leu-Asn-Gln-Ser-
Val-Glu-Ile-Asn-Cys-Thr-Arg-Pro-Asn-Asn-Asn-Thr-
Arg-Lys-Ser-Ile-Arg-Ile-Gln-Arg-Gly-Pro-Gly-Arg-

Aminosäuren 334-381 einschließlich, nämlich

Lys-Ile-Gly-
Asn-Met-Arg-Gln-Ala-His-Cys-Asn-Ile-Ser-Arg-Ala-
Lys-Trp-Asn-Ala-Thr-Leu-Lys-Gln-Ile-Ala-Ser-Lys-
Leu-Arg-Glu-Gln-Phe-Gly-Asn-Asn-Lys-Thr-Ile-Ile-
Phe-Lys-Gln-Ser-Ser-Gly-Gly-Asp-Pro-

Aminosäuren 397-424 einschließlich, nämlich
Cys-Asn-Ser-

Thr-Gln-Leu-Phe-Asn-Ser-Thr-Trp-Phe-Asn-Ser-
Thr-Trp-Ser-Thr-Glu-Gly-Ser-Asn-Asn-Thr-Glu-
Gly-Ser-Asp-

Aminosäuren 466-500 einschließlich, nämlich

Leu-Thr-Arg-
Asp-Gly-Gly-Asn-Asn-Asn-Asn-Gly-Ser-Glu-Ile-Phe-
Arg-Pro-Gly-Gly-Gly-Asp-Met-Arg-Asp-Asn-Trp-Arg-
Ser-Glu-Leu-Tyr-Lys-Tyr-Lys-Val-

Aminosäuren 510-523 einschließlich, nämlich

Pro-Thr-Lys-
Ala-Lys-Arg-Arg-Val-Val-Gln-Arg-Glu-Lys-Arg-

Aminosäuren 551-577 einschließlich, nämlich

Val-Gln-Ala-
Arg-Gln-Leu-Leu-Ser-Gly-Ile-Val-Gln-Gln-Gln-Asn-
Asn-Leu-Leu-Arg-Ala-Ile-Glu-Ala-Gln-Gln-His-Leu-

Aminosäuren 594-603 einschließlich, nämlich

Ala-Val-Glu-
Arg-Tyr-Leu-Lys-Asp-Gln-Gln-

Aminosäuren 621-630 einschließlich, nämlich

Pro-Trp-Asn-
Ala-Ser-Trp-Ser-Asn-Lys-Ser-

Aminosäuren 657-679 einschließlich, nämlich

Leu-Ile-Glu-
Glu-Ser-Gln-Asn-Gln-Gln-Glu-Lys-Asn-Glu-Gln-
Glu-Leu-Leu-Glu-Leu-Asp-Lys-Trp-Ala-

Aminosäuren 719-758 einschließlich, nämlich

Arg-Val-Arg-
Gln-Gly-Tyr-Ser-Pro-Leu-Ser-Phe-Gln-Thr-His-
Leu-Pro-Thr-Pro-Arg-Gly-Pro-Asp-Arg-Pro-Glu-
Gly-Ile-Glu-Glu-Glu-Gly-Gly-Glu-Arg-Asp-Arg-
Asp-Arg-Ser-Ile-

Aminosäuren 780-803 einschließlich, nämlich

Tyr-His-
Arg-Leu-Arg-Asp-Leu-Leu-Leu-Ile-Val-Thr-Arg-
Ile-Val-Glu-Leu-Leu-Gly-Arg-Arg-Gly-Trp-Glu-

7. Gereinigtes Peptid nach den Ansprüchen 3 und 4, dadurch gekennzeichnet, daß es ausgewählt ist aus der folgenden Gruppe, wobei die erste und letzte Aminosäure jeweils der N-terminalen bzw. C-terminalen Aminosäure entsprechen,
Aminosäuren 127-183 einschließlich, nämlich

Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

Aminosäuren 197-201, einschließlich, nämlich

Leu-Asp-Ile-Ile-Pro-Ile-Asp-Asn-Asp-Thr-Thr-

Aminosäuren 239-294 einschließlich, nämlich

Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

Aminosäuren 300-327 einschließlich, nämlich

Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

Aminosäuren 334-381 einschließlich, nämlich

Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

Aminosäuren 397-424 einschließlich, nämlich

Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

Aminosäuren 466-500 einschließlich, nämlich

Leu-Thr-Arg-

Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

Aminosäuren 621-630 einschließlich, nämlich

Pro-Trp-Asn-

Ala-Ser-Trp-Ser-Asn-Lys-Ser-

8. Gereinigtes Peptid nach einem der Ansprüche 3 oder 7, dadurch gekennzeichnet, daß es ein Epitop-tragendes Peptid ist und insbesondere, daß es ein immunogenes Peptid ist.

9. Gereinigtes Peptid nach Anspruch 8, in einer oligomeren Form, insbesondere ein wasserlösliches immunogenes Oligomer.

10. Hybridpolypeptid, umfassend ein Peptid nach einem der Ansprüche 3, 4, 7 oder 8 und mit N-terminalen und C-terminalen Enden, die kovalent an andere Aminosäuren gebunden sind als an die, die normalerweise mit diesen im gp110 von LAV assoziiert sind, wobei die letztgenannten Aminosäuren dann frei sind oder zu einer anderen Polypeptidsequenz gehören, insbesondere einer Sequenz mit einer Größe, die ausreicht, um eine verbesserte Immunogenität bereitzustellen.

11. Zusammensetzung von Peptiden, dadurch gekennzeichnet, daß sie mindestens zwei Peptide nach einem der Ansprüche 1 bis 8 umfaßt.

12. Immunogene Zusammensetzung, umfassend ein gereinigtes Peptid nach Anspruch 1, 2, 7 oder 8, oder ein gereinigtes Oligomer nach Anspruch 9, oder ein Hybridpolypeptid nach Anspruch 10 und einen geeigneten pharmazeutischen Träger.

13. DNA-Sequenz, dadurch gekennzeichnet, daß sie ein Peptid nach einem der Ansprüche 1 bis 8 codiert.

14. DNA-Sequenz nach Anspruch 13, dadurch gekennzeichnet, daß sie in einen Vektor eingebaut ist.

15. Verfahren zur chemischen Synthese eines Peptids oder Polypeptids nach einem der Ansprüche 1 bis 8, gekennzeichnet durch:
aufeinanderfolgende Kondensation entweder von aufeinanderfolgenden Aminosäuren in Zweiergruppen in der richtigen Ordnung oder aufeinanderfolgenden Peptidfragmenten, die vorher zugänglich waren oder gebildet wurden und schon einige Aminoacylreste in der geeigneten Reihenfolge enthalten, wobei alle reaktiven Gruppen außer den Carboxyl- und Aminogruppen, die an der Bildung der Peptidbindung beteiligt sind, geschützt sind,
gegebenenfalls Aktivierung der Carboxylgruppen vor der Bildung der Peptidbindung,
Gewinnung des gebildeten Peptids oder Polypeptids.

16. Verfahren zur Herstellung eines Peptids nach einem der Ansprüche 1 bis 8, gekennzeichnet durch:
Transformation eines prokaryontischen oder eukaryontischen Wirtes mit einem geeigneten Vektor, der vorher durch die entsprechende DNA gemäß Anspruch 13 modifiziert wurde, unter Bedingungen, die die Produktion des gewünschten Peptids ermöglichen,
Gewinnung und Reinigung des so erhaltenen Peptids, beispielsweise durch Elektrophorese.

17. Verfahren zur in vitro-Diagnose des Vorliegens von anti-LAV-Antikörpern in biologischen Medien, dadurch gekennzeichnet, daß es das Inkontaktbringen der biologischen Flüssigkeit mit dem Produkt eines der Ansprüche 1 bis 8 unter Bedingungen umfaßt, die für die Bildung eines Komplexes zwischen den Antikörpern und dem Produkt geeignet sind, und Nachweis des Komplexes als Hinweis auf die Gegenwart der Antikörper in der biologischen Flüssigkeit.

EP 0 387 914 B1

18. Kit für die in vitro-Diagnose der Gegenwart von Antikörpern gegen LAV-Virus in Flüssigkeiten, umfassend:
ein Peptid nach einem der Ansprüche 1 bis 8 oder eine Zusammensetzung nach Anspruch 11,
biologische und chemische Reagenzien, die zur Durchführung des diagnostischen Tests notwendig sind,
z.B. geeignete Puffer, anti-menschliche Immunglobuline, die vorher mit einer Fluoreszenzmolekül oder
einem Enzym markiert wurden,
wenn die anti-menschlichen Immunglobuline mit einem Enzym markiert wurden, ein Substrat, das durch
das Enzym hydrolysierbar ist und ein Signal liefert, das die Gegenwart eines Antikörpers in der biologischen Flüssigkeit anzeigt.

19. Verfahren zur Herstellung von monoclonalen Antikörpern gegen ein gereinigtes Peptid nach einem der
Ansprüche 1 bis 9, umfassend die folgenden Schritte:
- Fusion von Myelomzellen eines vorher mit diesem gereinigten Peptid immunisierten Tieres mit
Splenocyten zum Erhalt von Hybridomen;
- Auswahl der Hybridomen, die die Antikörper mit Spezifität für das gereinigte Peptid produzieren,
- Gewinnung der Antikörper gegen das gereinigte Peptid.

20. Antikörper, dadurch gekennzeichnet, daß er durch das gereinigte Peptid nach einem der Ansprüche 1 bis
9 erkannt wird.

21. Antikörper nach Anspruch 20, dadurch gekennzeichnet, daß es ein monoclonaler Antikörper ist.

22. Verwendung von Antikörpern nach Anspruch 20 oder 21, zur Identifizierung des Hüllglycoproteins des
LAV-Virus, oder auch für die Bestimmung des relativen Anteils dieses Antigens in einer biologischen Probe, die LAV oder verwandte Viren enthält.

**Patentansprüche für folgenden Vertragsstaat :AT**

1. Verfahren zur Herstellung eines gereinigten Peptids, herausschneidbar aus einem gereinigten
Hüllglycoprotein des LAV-Virus mit einem Molekulargewicht im Bereich von 110 000, meßbar durch seinen
Wanderungsabstand in einem Wanderungssystem im Vergleich mit den Abständen von Standardproteinen mit bekannten Molekulargewichten im gleichen Wanderungssystem, wobei das Glycoprotein nach Behandlung mit Endoglycosidasen und nachfolgender Entfernung der Zuckerreste ein Protein mit einem Molekulargewicht von etwa 90 000 Dalton liefert, meßbar im gleichen Wanderungssystem, oder aus einem
Polypeptid, das das gleiche Polypeptidgrundgerüst wie obiges Glycoprotein hat, wobei das Peptid eine
Größe aufweist, die 200 Aminosäuren nicht überschreitet, gekennzeichnet durch
Transformation eines prokaryontischen oder eukaryontischen Wirtes mit einem geeigneten Vektor, der
vorher durch die entsprechende DNA modifiziert wurde, unter Bedingungen, die die Produktion des gewünschten Peptids ermöglichen,
Gewinnung und Reinigung des so erhaltenen Peptids, beispielsweise durch Elektrophorese.

2. Verfahren zur Herstellung eines gereinigten Peptids, dadurch gekennzeichnet, daß sich das gewonnene
Peptid von einem gereinigten Peptid nach Anspruch 1 unterscheidet, wobei es im wesentlichen die gleichen immunologischen oder immunogenen Eigenschaften, wie das entsprechende nicht-modifizierte
Peptid aufweist, wobei solche Peptide beispielsweise eine oder mehrere Aminosäuren aufweisen, die von
denen eines bestimmten Peptids nach Anspruch 1 verschieden sind.

3. Verfahren zur Herstellung eines gereinigten Peptids nach Anspruch 1 oder 2, dadurch gekennzeichnet,
daß das gewonnene Peptid mindestens ein neutralisierendes Epitop enthält, wobei ein solches Epitop insbesondere eine Aminosäuresequenz mit der Formel Asn-X-Ser oder Asn-X-Thr umfaßt, wobei X jede andere mögliche Aminosäure ist.

4. Verfahren zur Herstellung eines gereinigten Peptids nach Anspruch 1 oder 2, dadurch gekennzeichnet,
daß das gewonnene Peptid mindestens ein neutralisierendes Epitop enthält, wobei ein solches Epitop insbesondere eine Aminosäuresequenz mit der Formel Asn-X-Ser oder Asn-X-Thr umfaßt, wobei X jede andere mögliche Aminosäure ist und das Epitop glycosyliert ist oder nicht.

5. Verfahren zur Herstellung eines gereinigten Peptids nach einem der Ansprüche 1 bis 4, dadurch gekennzeichnet, daß das gewonnene Peptid von der Nucleotidsequenz codiert wird, die sich jeweils zwischen
den folgenden Positionen erstreckt:

a) Nucleotide 6171 bis 6276 mit der folgenden Aminosäuresequenz

**AsnAlaThrAsnThrAsnSerSerAsnThrAsnSerSerSerGlyGluMetMet MetGluLysGlyGluIleLysAsnCysSerPheAsnIleSerThrSerIle**

oder
b) Nucleotide 6337 bis 6387 mit der folgenden Aminosäuresequenz

**AsnAspThrThrSerTyrThrLeuThrSerCysAsnThrSerValIleThr**

c) Nucleotide 6466 bis 6516 mit der folgenden Aminosäuresequenz

**AsnAsnLysThrPheAsnGlyThrGlyProCysThrAsnValSerThrVal**

oder
d) Nucleotide 6562 bis 6696 mit der folgenden Aminosäuresequenz

**LeuAsnGlySerLeuAlaGluGluGluValValIleArgSerAlaAsnPheThr AspAsnAlaLysThrIleIleValGlnLeuAsnGlnSerValGluIleAsnCys ThrArgProAsnAsnAsnThrArgLys,**

oder
e) Nucleotide 6937 bis 7005 mit der folgenden Aminosäuresequenz

**AsnSerThrGlnLeuPheAsnSerThrTrpPheAsnSerThrTrpSerThrGlu GlySerAsnAsnThr,**

oder
f) Nucleotide 7612 bis 7746 mit der folgenden Aminosäuresequenz:

**AsnAlaSerTrpSerAsnLysSerLeuGluGlnIleTrpAsnAsnMetThrTrp MetGluTrpAspArgGluIleAsnAsnTyrThrSerLeuIleHisSerLeuIle GluGluSerGlnAsnGlnGlnGluLys**

6. Verfahren zur Herstellung eines gereinigten Peptids nach Anspruch 1 oder 2, dadurch gekennzeichnet, daß das gewonnene Peptid aus der folgenden Gruppe ausgewählt ist, wobei die erste und letzte Aminosäure jeweils der N-terminalen bzw. C-terminalen Aminosäure entsprechen,
Aminosäuren 8-23 einschließlich, nämlich

**Met-Arg-Val-Lys-**

**Glu-Lys-Tyr-Gln-His-Leu-Trp-Arg-Trp-Gly-Trp-Lys-**

Aminosäuren 63-78 einschließlich, nämlich

**Ser-Asp-Ala-Lys-**

**Ala-Tyr-Asp-Thr-Glu-Val-His-Asn-Val-Trp-Ala-Thr-**

Aminosäuren 82-90 einschließlich, nämlich

31

Val-Pro-Thr-Asp-Pro-Asn-Pro-Gln-Glu-

Aminosäuren 97-123 einschließlich, nämlich

Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-

Aminosäuren 127-183 einschließlich, nämlich

Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

Aminosäuren 191-201 einschließlich, nämlich

Leu-Asp-Ile-

Ile-Pro-Ile-Asp-Asn-Asp-Thr-Thr-

Aminosäuren 239-294 einschließlich, nämlich

Lys-Cys-Asn-

Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

Aminosäuren 300-327 einschließlich, nämlich

Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

Aminosäuren 334-381 einschließlich, nämlich

```
                                              Lys-Ile-Gly-
    Asn-Met-Arg-Gln-Ala-His-Cys-Asn-Ile-Ser-Arg-Ala-
    Lys-Trp-Asn-Ala-Thr-Leu-Lys-Gln-Ile-Ala-Ser-Lys-
    Leu-Arg-Glu-Gln-Phe-Gly-Asn-Asn-Lys-Thr-Ile-Ile-
    Phe-Lys-Gln-Ser-Ser-Gly-Gly-Asp-Pro-
```

Aminosäuren 397-424 einschließlich, nämlich

```
                                            Cys-Asn-Ser-
    Thr-Gln-Leu-Phe-Asn-Ser-Thr-Trp-Phe-Asn-Ser-
    Thr-Trp-Ser-Thr-Glu-Gly-Ser-Asn-Asn-Thr-Glu-
    Gly-Ser-Asp-
```

Aminosäuren 466-500 einschließlich, nämlich

```
                                            Leu-Thr-Arg-
    Asp-Gly-Gly-Asn-Asn-Asn-Asn-Gly-Ser-Glu-Ile-Phe-
    Arg-Pro-Gly-Gly-Gly-Asp-Met-Arg-Asp-Asn-Trp-Arg-
    Ser-Glu-Leu-Tyr-Lys-Tyr-Lys-Val-
```

Aminosäuren 510-523 einschließlich, nämlich

```
                                            Pro-Thr-Lys-
       Ala-Lys-Arg-Arg-Val-Val-Gln-Arg-Glu-Lys-Arg-
```

Aminosäuren 551-577 einschließlich, nämlich

```
                                            Val-Gln-Ala-
    Arg-Gln-Leu-Leu-Ser-Gly-Ile-Val-Gln-Gln-Gln-Asn-
    Asn-Leu-Leu-Arg-Ala-Ile-Glu-Ala-Gln-Gln-His-Leu-
```

Aminosäuren 594-603 einschließlich, nämlich

```
                                            Ala-Val-Glu-
       Arg-Tyr-Leu-Lys-Asp-Gln-Gln-
```

Aminosäuren 621-630 einschließlich, nämlich

```
                                            Pro-Trp-Asn-
       Ala-Ser-Trp-Ser-Asn-Lys-Ser-
```

Aminosäuren 657-679 einschließlich, nämlich

```
                                        Leu-Ile-Glu-
Glu-Ser-Gln-Asn-Gln-Gln-Glu-Lys-Asn-Glu-Gln-
Glu-Leu-Leu-Glu-Leu-Asp-Lys-Trp-Ala-
```

Aminosäuren 719-758 einschließlich, nämlich

```
                                        Arg-Val-Arg-
Gln-Gly-Tyr-Ser-Pro-Leu-Ser-Phe-Gln-Thr-His-
Leu-Pro-Thr-Pro-Arg-Gly-Pro-Asp-Arg-Pro-Glu-
Gly-Ile-Glu-Glu-Glu-Gly-Gly-Glu-Arg-Asp-Arg-
Asp-Arg-Ser-Ile-
```

Aminosäuren 780-803 einschließlich, nämlich

```
                                        Tyr-His-
Arg-Leu-Arg-Asp-Leu-Leu-Leu-Ile-Val-Thr-Arg-
Ile-Val-Glu-Leu-Leu-Gly-Arg-Arg-Gly-Trp-Glu-
```

7. Verfahren zur Herstellung eines gereinigten Peptids nach den Ansprüchen 3 und 4, dadurch gekennzeichnet, daß das gewonnene Peptid ausgewählt ist aus der folgenden Gruppe, wobei die erste und letzte Aminosäure jeweils der N-terminalen bzw. C-terminalen Aminosäure entsprechen,
Aminosäuren 127-183 einschließlich, nämlich

```
                                        Val-Lys-Leu-Thr-
Pro-Leu-Cys-Val-Ser-Leu-Lys-Cys-Thr-Asp-Leu-Gly-
Asn-Ala-Thr-Asn-Thr-Asn-Ser-Ser-Asn-Thr-Asn-Ser-
Ser-Ser-Gly-Glu-Met-Met-Met-Glu-Lys-Gly-Glu-Ile-
Lys-Asn-Cys-Ser-Phe-Asn-Ile-Ser-Thr-Ser-Ile-Arg-
Gly-Lys-Val-Gln-Lys-
```

Aminosäuren 197-201, einschließlich, nämlich

```
                                        Leu-Asp-Ile-
Ile-Pro-Ile-Asp-Asn-Asp-Thr-Thr-
```

Aminosäuren 239-294 einschließlich, nämlich

```
                                        Lys-Cys-Asn-
Asn-Lys-Thr-Phe-Asn-Gly-Thr-Gly-Pro-Cys-Thr-Asn-
Val-Ser-Thr-Val-Gln-Cys-Thr-His-Gly-Ile-Arq-Pro-
Val-Val-Ser-Thr-Gln-Leu-Leu-Leu-Asn-Gly-Ser-Leu-
Ala-Glu-Glu-Glu-Val-Val-Ile-Arg-Ser-Ala-Asn-Phe-
Thr-Asp-Asn-Ala-Lys-
```

Aminosäuren 300-327 einschließlich, nämlich

Leu-Asn-Gln-Ser-
Val-Glu-Ile-Asn-Cys-Thr-Arg-Pro-Asn-Asn-Asn-Thr-
Arg-Lys-Ser-Ile-Arg-Ile-Gln-Arg-Gly-Pro-Gly-Arg-

Aminosäuren 334-381 einschließlich, nämlich

Lys-Ile-Gly-
Asn-Met-Arg-Gln-Ala-His-Cys-Asn-Ile-Ser-Arg-Ala-
Lys-Trp-Asn-Ala-Thr-Leu-Lys-Gln-Ile-Ala-Ser-Lys-
Leu-Arg-Glu-Gln-Phe-Gly-Asn-Asn-Lys-Thr-Ile-Ile-
Phe-Lys-Gln-Ser-Ser-Gly-Gly-Asp-Pro-

Aminosäuren 397-424 einschließlich, nämlich

Cys-Asn-Ser-
Thr-Gln-Leu-Phe-Asn-Ser-Thr-Trp-Phe-Asn-Ser-
Thr-Trp-Ser-Thr-Glu-Gly-Ser-Asn-Asn-Thr-Glu-
Gly-Ser-Asp-

Aminosäuren 466-500 einschließlich, nämlich

Leu-Thr-Arg-
Asp-Gly-Gly-Asn-Asn-Asn-Asn-Gly-Ser-Glu-Ile-Phe-
Arg-Pro-Gly-Gly-Gly-Asp-Met-Arg-Asp-Asn-Trp-Arg-
Ser-Glu-Leu-Tyr-Lys-Tyr-Lys-Val-

Aminosäuren 621-630 einschließlich, nämlich

Pro-Trp-Asn-
Ala-Ser-Trp-Ser-Asn-Lys-Ser-

8. Verfahren zur Herstellung eines gereinigten Peptids nach einem der Ansprüche 3 oder 7, dadurch gekennzeichnet, daß das gewonnene Peptid ein Epitop-tragendes Peptid ist und insbesondere, daß es ein immunogenes Peptid ist.

9. Verfahren zur Herstellung eines gereinigten Peptids nach Anspruch 8, dadurch gekennzeichnet, daß das gewonnene Peptid dann in eine oligomere Form gebracht wird, insbesondere ein wasserlösliches immunogenes Oligomer.

10. Verfahren zur Herstellung eines Hybridpolypeptids, umfassend ein Peptid erhalten nach einem der Ansprüche 3, 4, 7 oder 8 und mit N-terminalen und C-terminalen Enden, wobei das Peptid kovalent an andere Aminosäuren gebunden wird als an die, die normalerweise mit diesen im gp110 von LAV assoziiert sind, wobei die letztgenannten Aminosäuren dann frei sind oder zu einer anderen Polypeptidsequenz gehören, insbesondere einer Sequenz mit einer Größe, die ausreicht, um eine verbesserte Idmmunogenität bereitzustellen.

11. Verfahren zur Herstellung einer Zusammensetzung von Peptiden, dadurch gekennzeichnet, daß man mindestens zwei Peptide nach einem der Ansprüche 1 bis 8 mischt.

12. Verfahren zur Herstellung einer immunogenen Zusammensetzung, umfassend ein gereinigtes Peptid erhalten nach Anspruch 1, 2, 7 oder 8, oder ein gereinigtes Oligomer nach Anspruch 9, oder ein Hybridpolypeptid nach Anspruch 10 gemischt mit einem geeigneten pharmazeutischen Träger.

13. Verfahren zur Herstellung einer DNA-Sequenz, gekennzeichnet durch
    - Spaltung der gesamten, dem vollständigem Genom einer LVA-Art entsprechenden DNA durch Verdauung mit einem geeigneten Restriktionsenzym,
    - Gewinnung des relevanten Fragments, das ein Peptid erhalten nach einem der Ansprüche 1 bis 8 codiert.

14. Verfahren zur Herstellung einer DNA-Sequenz nach Anspruch 13, dadurch gekennzeichnet, daß die DNA-Sequenz in einen Vektor eingebaut wird.

15. Verfahren zur chemischen Synthese eines Peptids oder Polypeptids nach einem der Ansprüche 1 bis 8, gekennzeichnet durch:
    aufeinanderfolgende Kondensation entweder von aufeinanderfolgenden Aminosäuren in Zweiergruppen in der richtigen Ordnung oder aufeinanderfolgenden Peptidfragmenten, die vorher zugänglich waren oder gebildet wurden und schon einige Aminoacylreste in der geeigneten Reihenfolge enthalten, wobei alle reaktiven Gruppen außer den Carboxyl- und Aminogruppen, die an der Bildung der Peptidbindung beteiligt sind, geschützt sind,
    gegebenenfalls Aktivierung der Carboxylgruppen vor der Bildung der Peptidbindung,
    Gewinnung des gebildeten Peptids oder Polypeptids.

16. Verfahren zur in vitro-Diagnose des Vorliegens von anti-LAV-Antikörpern in biologischen Medien, dadurch gekennzeichnet, daß es das Inkontaktbringen der biologischen Flüssigkeit mit dem Produkt eines der Ansprüche 1 bis 8 unter Bedingungen umfaßt, die für die Bildung eines Komplexes zwischen den Antikörpern und dem Produkt geeignet sind, und Nachweis des Komplexes als Hinweis auf die Gegenwart der Antikörper in der biologischen Flüssigkeit.

17. Verfahren zur Herstellung von monoclonalen Antikörpern gegen ein gereinigtes Peptid erhalten nach einem der Ansprüche 1 bis 9, umfassend die folgenden Schritte:
    - Fusion von Myelomzellen eines vorher mit diesem gereinigten Peptid immunisierten Tieres mit Splenocyten zum Erhalt von Hybridomen;
    - Auswahl der Hybridomen, die die Antikörper mit Spezifität für das gereinigte Peptid produzieren,
    - Gewinnung der Antikörper gegen das gereinigte Peptid.

18. Verfahren zur Herstellung von Antikörpern, gekennzeichnet durch die Immunisierung eines Tieres mit einem gereinigten Peptid erhalten nach den Ansprüchen 1 bis 9, Gewinnung der Antikörper, die durch das gereinigte Peptid nach einem der Ansprüche 1 bis 9 erkannt werden.

19. Verwendung von Antikörpern erhalten nach Anspruch 17 oder 18, zur Identifizierung des Hüllglycoproteins des LAV-Virus, oder auch für die Bestimmung des relativen Anteils dieses Antigens in einer biologischen Probe, die LAV oder verwandte Viren enthält.

## Revendications

### Revendications pour les Etats contractants suivants : BE, CH, DE, FR, GB, IT, LI, LU, NL, SE

1. Peptide purifié extractible d'une glycoprotéine purifiée à partir de l'enveloppe du virus LAV, d'un poids moléculaire de l'ordre de 110.000, évalué d'après sa distance de migration dans un système de migration par comparaison avec les distances de migration de protéines standard, ayant des poids moléculaires connus, mesurés dans le même système de migration, ladite glycoprotéine, après traitement avec des endoglycosidases et élimination des résidus glucidiques, restituant une protéine d'un poids moléculaire de 90.000 daltons environ, mesuré dans le même système de migration, ou obtenue à partir d'un polypeptide ayant le même squelette peptidique que ladite glycoprotéine, la taille de ce peptide ne dépassant pas 200 acides aminés,

2. Peptide purifié caractérisé en ce qu'il diffère d'un peptide purifié selon la revendication 1, tout en possèdant des propriétés immunologiques ou immunogéniques substantiellement identiques à celles du peptide correspondant non-modifié, par exemple, par un ou plusieurs acides aminés autres que ceux d'un peptide défini, selon la revendication 1.

3. Peptide purifié selon les revendications 1 ou 2, caractérisé en ce qu'il contient au moins un épitope neutralisant, ledit épitope comprenant plus particulièrement une séquence d'acides aminés de formule Asn-X-Ser ou Asn-X-Thr, où X représente tout autre acide aminé.

4. Peptide purifié selon les revendications 1 ou 2, caractérisé en ce qu'il contient au moins un épitope neutralisant, ledit épitope comprenant plus particulièrement une séquence d'acides aminés de formule Asn-X-Ser ou Asn-X-Thr, où X représente tout autre acide aminé possible et l'épitope peut être glycosylé ou non.

5. Peptide purifié selon l'une quelconque des revendication de 1 à 4 caractérisé en ce qu'il est codé par la séquence nucléotidique qui s'étend respectivement entre les positions suivantes :
a) nucléotides compris entre 6171 et 6276 pour un peptide de séquence d'acides aminés suivante :

AsnAlaThrAsnThrAsnSerSerAsnThrAsnSerSerSerGlyGluMetMetMetGluLys

GlyGluIleLysAsnCysSerPheAsnIleSerThrSerIle

ou
b) nucléotides compris entre 6337 et 6387 pour un peptide de séquence d'acides aminés suivante :
AsnAspThrThrSerTyrThrLeuThrSerCysAsnThrSerValIleThr

c) nucléotides compris entre 6466 et 6516 pour un peptide de séquence d'acides aminés suivante :
AsnAsnLysThrPheAsnGlyThrGlyProCysThrAsnValSerThrVal

ou
d) nucléotides compris entre 6562 et 6696 pour un peptide de séquence d'acides aminés suivante :

LeuAsnGlySerLeuAlaGluGluGluValValIleArgSerAlaAsnPheThrAspAsnAla

LysThrIleIleValGlnLeuAsnGlnSerValGluIleAsnCysThrArgProAsnAsnAsnThr

ArgLys

ou
e) nucléotides compris entre 6937 et 7005 pour un peptide de séquence d'acides aminés suivante :

AsnSerThrGlnLeuPheAsnSerThrTrpPheAsnSerThrTrpSerThrGluGlySerAsn

AsnThr

ou
d) nucléotides compris entre 7612 et 7746 pour un peptide de séquence d'acides aminés suivante :

AsnAlaSerTrpSerAsnLysSerLeuGluGlnIleTrpAsnAsnMetThrTrpMetGluTrp

AspArgGluIleAsnAsnTyrThrSerLeuIleHisSerLeuIleGluGluSerGlnAsnGlnGln

GluLys

6. Peptide purifié selon les revendications 1 ou 2, caractérisé en ce qu'il est choisi à partir du groupe suivant, le premier et le dernier acide aminé correspondant respectivement aux acides aminés N-terminal et C-terminal

acides aminés 8-23 compris, par exemple, Met-Arg-Val-Lys-Glu-Lys-Tyr-Gln-His-Leu-Trp-Arg-Trp-Gly-Trp-Lys-

acides aminés 63-78 compris, par exemple, Ser-Asp-Ala-Lys-Ala-Tyr-Asp-Thr-Glu-Val-His-Asn-Val-Trp-Ala-Thr-

acides aminés 82-90 compris, par exemple, Val-Pro-Thr-Asp-Pro-Asn-Pro-Gln-Glu-

acides aminés 97-123 compris, par exemple, Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-

acides aminés 127-183 compris, par exemple, Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

acides aminés 191-201 compris, par exemple, Leu-Asp-Ile-Ile-Pro-Ile-Asp-Asn-Asp-Thr-Thr-

acides aminés 239-294 compris, par exemple, Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

acides aminés 300-327 compris, par exemple, Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

acides aminés 334-381 compris, par exemple, Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

acides aminés 397-424 compris, par exemple, Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

acides aminés 466-500 compris, par exemple, Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

acides aminés 510-523 compris, par exemple, Pro-Thr-Lys-Ala-Lys-Arg-Arg-Val-Val-Gln-Arg-Glu-Lys-Arg-

acides aminés 551-577 compris, par exemple, Val-Gln-Ala-Arg-Gln-Leu-Leu-Ser-Gly-Ile-Val-Gln-Gln-Gln-Asn-Asn-Leu-Leu-Arg-Ala-Ile-Glu-Ala-Gln-Gln-His-Leu-

acides aminés 594-603 compris, par exemple, Ala-Val-Glu-Arg-Tyr-Leu-Lys-Asp-Gln-Gln-

acides aminés 621-630 compris, par exemple, Pro-Trp-Asn-Ala-Ser-Trp-Ser-Asn-Lys-Ser-

acides aminés 657-679 compris, par exemple, Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-

acides aminés 719-758 compris, par exemple, Arg-Val-Arg-Gln-Gly-Tyr-Ser-Pro-Leu-Ser-Phe-Gln-Thr-His-Leu-Pro-Thr-Pro-Arg-Gly-Pro-Asp-Arg-Pro-Glu-Gly-Ile-Glu-Glu-Glu-Gly-Gly-Glu-Arg-Asp-Arg-Asp-Arg-Ser-Ile-

acides aminés 780-803 compris, par exemple, Tyr-His-Arg-Leu-Arg-Asp-Leu-Leu-Leu-Ile-Val-Thr-Arg-Ile-Val-Glu-Leu-Leu-Gly-Arg-Arg-Gly-Trp-Glu

7. Peptide purifié selon les revendication 3 et 4, caractérisé en ce qu'il est choisi à partir du groupe suivant, le premier et le dernier acide aminé correspondant respectivement aux acides aminés N-terminal et C-terminal

acides aminés 127-183 compris, par exemple, Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

acides aminés 197-201 compris, par exemple, Leu-Asp-Ile-Ile-Pro-Ile-Asp-Asn-Asp-Thr-Thr-

acides aminés 239-294 compris, par exemple, Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

acides aminés 300-327 compris, par exemple, Leu-Asn-Gln-Ser-Val-Glu-Ile-Asn-Cys-Thr-Arg--Pro-Asn-Asn-Asn-Thr-Arg-Lys-Ser-Ile-Arg-Ile-Gln-Arg-Gly-Pro-Gly-Arg-

acides aminés 334-381 compris, par exemple, Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

acides aminés 397-424 compris, par exemple, Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

acides aminés 466-500 compris, par exemple, Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

acides aminés 621-630 compris, par exemple, Pro-Trp-Asn-Ala-Ser-Trp-Ser-Asn-Lys-Ser

8. Peptide purifié selon l'une quelconque des revendications 3 ou 7, caractérisé en ce qu'il porte un épitope et particulièrement, en ce qu'il s'agit d'un peptide immunogène.

9. Peptide purifié selon la revendication 8, sous forme d'un oligomère, plus particulièrement, d'un oligomère immunogène, soluble dans l'eau,

10. Polypeptide hybride comprenant un peptide selon l'une quelconque des revendications 3, 4, 7 ou 8 et ayant des extrémités N-terminal ou C-terminal, liées par une liaison covalent à des acides aminés autres que ceux qui leur sont normalement associés dans le gp110 du LAV, lesquels acides aminés cités en dernier sont alors libres ou attachés à une autre séquence polypeptidique, particulièrement à une séquence d'une longueur suffisante pour présenter une immunogénicité supérieure.

11. Composition de peptides caractérisée en ce qu'elle comprend au moins 2 peptides selon l'une quelconque des revendications de 1 à 8.

12. Composition immunogène comprenant un peptide purifié selon les revendications 1, 2, 7 ou 8 ou un oligomères purifié selon la revendication 9 ou un polypeptide hybride selon la revendication 10 et un vecteur pharmaceutique approprié.

13. Séquence d'ADN caractérisée en ce qu'elle code pour un peptide selon l'une quelconque des revendications de 1 à 8.

14. Séquence d'ADN selon la revendication 13 caractérisée en ce qu'elle est insérée dans un vecteur

15. Procédé de synthèse chimique d'un peptide ou d'un polypeptide selon l'une des revendications de 1 à 8 caractérisé en ce que :
    - les acides aminés sont condensés successivement par paire dans l'ordre approprié, ou séquentiellement des fragments peptidiques obtenus préalablement ou formés et contenant déjà certains résidus aminoacylés dans l'ordre approprié respectivement, tous les groupes réactifs, autres que les groupes carboxyle ou amino, qui participeront à la formation de la liaison peptidique étant protégés,
    - les groupes carboxyle subissent une activation possible, avant la formation de la liaison peptidique,
    - le peptide ou le polypeptide formé sont récupérés.

16. Procédé de préparation d'un peptide selon l'une quelconque des revendications de 1 à 8, caractérisé en ce que :
    - un hôte procaryote ou eucaryote est transformé à l'aide d'un vecteur approprié préalablement modifié par l'ADN correspondant, selon la revendication 13 dans des conditions permettant de produire le peptide voulu,
    - le peptide ainsi obtenu est récupéré et purifié, par exemple, par électrophorèse.

17. Procédé de diagnostic in vitro de la présence des anticorps anti-LAV dans des milieux biologiques, caractérisé par la mise en contact desdits fluides biologiques avec le produit de l'une quelconque des revendications de 1 à 8 dans des conditions permettant la formation d'un complexe entre lesdits anticorps et ledit produit, et la révélation dudit complexe comme indicateur de la présence desdits anticorps dans lesdits fluides biologiques.

18. Trousse pour le diagnostic in vitro de la présence des anticorps dirigés contre le virus LAV dans des fluides, la dite trousse comprenant :
    . un peptide selon l'une quelconque des revendications de 1 à 8 ou une composition selon la revendication 11,

.  un réactif biologique ou chimique nécessaire à la mise en oeuvre du test diagnostique, tels que des tampons adéquats, des immunoglobulines antihumaines, préalablement marquées par une molécule fluorescente ou par une enzyme,

-  lorsque les immunoglobulines anti-humaines sont marquées par une enzyme, un substrat hydrolysable par l'enzyme, produisant un signal indicateur de la présence d'anticorps dans le fluide biologique.

19.  Procédé de production d'anticorps monoclonaux dirigés contre le peptide purifié selon l'une quelconque des revendications de 1 à 9, comprenant les étapes suivantes :
-  fusion de cellules de myélome d'un animal immunisé préalablement par ce peptide purifié, avec des splenocytes pour obtenir des hybridomes;
-  sélection des hybridomes secrétant des anticorps spécifiques du peptide purifié;
-  récupération des anticorps dirigés contre le peptide purifié.

20.  Anticorps caractérisé en ce qu'il est reconnu par le peptide purifié selon l'une quelconque des revendications de 1 à 9.

21.  Anticorps selon la revendication 20 caractérisé en ce qu'il est un anticorps monoclonal.

22.  Utilisation d'anticorps selon les revendications 20 ou 21 pour l'identification de la glycoprotéine d'enveloppe du virus LAV ou même pour la détermination de la proportion relative de cet antigène dans un échantillon biologique renfermant le LAV ou des virus apparentés.

**Revendications pour l'Etat contractant suivant : AT**

1.  Procédé pour la préparation d'un peptide purifié, extractible d'une glycoprotéine d'enveloppe purifiée du virus LAV, d'un poids moléculaire de l'ordre de 110.000, mesurable selon sa distance de migration dans un système de migration, par comparaison avec les distances de protéines standard ayant des poids moléculaires connus, dans le même système de migration, ladite glycoprotéine restituant, après traitement par des endoglycosidases et élimination des résidus glucidiques, une protéine d'un poids moléculaire de 90.000 daltons environ, tel qu'on peut le mesurer dans le même système de migration, ou obtenu à partir d'un polypeptide ayant le même squelette peptidique que ladite glycoprotéine, ledit polypeptide ayant une taille ne dépassant pas 200 acides aminés, caractérisé en ce que :
-  l'hôte procaryote ou eucaryote est transformé à l'aide d'un vecteur convenable préalablement modifié par l'ADN correspondant dans des conditions permettant de produire le peptide voulu,
-  le peptide ainsi obtenu est récupéré et purifié, par exemple, par électrophorèse.

2.  Procédé pour la préparation d'un peptide purifié caractérisé en ce que le peptide récupéré est distinct d'un peptide purifié selon la revendication 1, tout en ayant substantiellement les mêmes propriétés immunologiques ou immunogéniques que le peptide correspondant non modifié, un tel peptide pouvant avoir, par exemple, un ou plusieurs acides aminés différents de ceux d'un peptide défini selon la revendication 1.

3.  Procédé pour la préparation d'un peptide purifié selon les revendications 1 et 2, caractérisé en ce que le peptide récupéré contient au moins un épitope neutralisant, ledit épitope comprenant plus particulièrement une séquence d'acides aminés de formule Asn-X-Ser ou Asn-X-Thr, où X représente tout autre acide aminé.

4.  Procédé pour la préparation d'un peptide purifié selon les revendications 1 ou 2 caractérisé en ce que le peptide récupéré contient un épitope neutralisant au moins, ledit épitope comprenant plus particulièrement une séquence d'acides aminés ayant la formule de Asn-X-Ser ou Asn-X-Thr où X correspond à tout autre acide aminé possible et l'épitope est glycosylé ou non.

5.  Procédé pour la préparation d'un peptide purifié selon l'une quelconque des revendications 1 à 4 caractérisé en ce que le peptide récupéré est codé par la séquence nucléotidique, située respectivement entre les positions suivantes :
a)  nucléotides de 6171 à 6276 pour un peptide de séquence d'acides aminés suivante

```
AsnAlaThrAsnThrAsnSerSerAsnThrAsnSerSerSerGlyGluMetMet
MetGluLysGlyGluIleLysAsnCysSerPheAsnIleSerThrSerIle
```

ou

b) nucléotides de 6337 et 6387 pour un peptide de séquence d'acides aminés suivante

```
AsnAspThrThrSerTyrThrLeuThrSerCysAsnThrSerValIleThr
```

c) nucléotides de 6466 et 6516 pour un peptide de séquence d'acides aminés suivante

```
AsnAsnLysThrPheAsnGlyThrGlyProCysThrAsnValSerThrVal
```

ou

d) nucléotides de 6562 et 6696 pour un peptide de séquence d'acides aminés suivante

```
LeuAsnGlySerLeuAlaGluGluGluValValIleArgSerAlaAsnPheThr
AspAsnAlaLysThrIleIleValGlnLeuAsnGlnSerValGluIleAsnCys
ThrArgProAsnAsnAsnThrArgLys,
```

ou

e) nucléotides de 6937 et 7005 pour un peptide de séquence d'acides aminés suivante

```
AsnSerThrGlnLeuPheAsnSerThrTrpPheAsnSerThrTrpSerThrGlu
GlySerAsnAsnThr,
```

ou

f) nucléotides de 7612 et 7746 pour un peptide de séquence d'acides aminés suivante

```
AsnAlaSerTrpSerAsnLysSerLeuGluGlnIleTrpAsnAsnMetThrTrp
MetGluTrpAspArgGluIleAsnAsnTyrThrSerLeuIleHisSerLeuIle
GluGluSerGlnAsnGlnGlnGluLys
```

6.  Procédé pour la préparation d'un peptide purifié selon les revendications 1 ou 2 caractérisé en ce que le peptide récupéré est choisi à partir du groupe suivant, le premier et dernier acide aminé correspondant respectivement aux acides aminés N-terminal et C-terminal,
acides aminés 8-23 compris, par exemple, Met-Arg-Val-Lys-Glu-Lys-Tyr-Gln-His-Leu-Trp-Alg-Trp-Gly-Trp-Lys-
acides aminés 63-78 compris, par exemple, Ser-Asp-Ala-Lys-Ala-Tyr-Asp-Thr-Glu-Val-His-Asn-Val-Trp-Ala-Thr-
acides aminés 82-90 compris, par exemple, Val-Pro-Thr-Asp-Pro-Asn-Pro-Gln-Glu-
acides aminés 97-123 compris, par exemple, Thr-Glu-Asn-Phe-Asn-Met-Trp-Lys-Asn-Asp-Met-Val-Glu-Gln-Met-His-Glu-Asp-Ile-Ile-Ser-Leu-Trp-Asp-Gln-Ser-Leu-
acides aminés 127-183 compris, par exemple, Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-
acides aminés 191-201 compris, par exemple, Leu-Asp-Ile-Ile-Pro-Ile-Asp-Asn-Asp-Thr-Thr-
acides aminés 239-294 compris, par exemple, Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-

41

Leu-Ala-Glu-Glu-Glu-Val-Val-Ile-Arg-Ser-Ala-Asn-Phe-Thr-Asp-Asn-Ala-Lys-

acides aminés 300-327 compris, par exemple, Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

acides aminés 334-381 compris, par exemple, Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

acides aminés 397-424 compris, par exemple, Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

acides aminés 466-500 compris, par exemple, Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

acides aminés 510-523 compris, par exemple, Pro-Thr-Lys-Ala-Lys-Arg-Arg-Val-Val-Gln-Arg-Glu-Lys-Arg-

acides aminés 551-577 compris, par exemple, Val-Gln-Ala-Arg-Gln-Leu-Leu-Ser-Gly-Ile-Val-Gln-Gln-Gln-Asn-Asn-Leu-Leu-Arg-Ala-Ile-Glu-Ala-Gln-Gln-His-Leu-

acides aminés 594-603 compris, par exemple, Ala-Val-Glu-Arg-Tyr-Leu-Lys-Asp-Gln-Gln-

acides aminés 621-630 compris, par exemple, Pro-Trp-Asn-Ala-Ser-Trp-Ser-Asn-Lys-Ser-

acides aminés 657-679 compris, par exemple, Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-

acides aminés 719-758 compris, par exemple, Arg-Val-Arg-Gln-Gly-Tyr-Ser-Pro-Leu-Ser-Phe-Gln-Thr-His-Leu-Pro-Thr-Pro-Arg-Gly-Pro-Asp-Arg-Pro-Glu-Gly-Ile-Glu-Glu-Glu-Gly-Gly-Glu-Arg-Asp-Arg-Asp-Arg-Ser-Ile-

acides aminés 780-803 compris, par exemple, Tyr-His-Arg-Leu-Arg-Asp-Leu-Leu-Leu-Ile-Val-Thr-Arg-Ile-Val-Glu-Leu-Leu-Gly-Arg-Arg-Gly-Trp-Glu-

7. Procédé pour la préparation d'un peptide purifié selon les revendications 3 et 4 caractérisé en ce que le peptide récupéré est choisi dans le groupe suivant, le premier et le dernier acide aminé correponsant respectivement aux acides aminés N-terminal et C-terminal

acides aminés 127-183 compris, par exemple, Val-Lys-Leu-Thr-Pro-Leu-Cys-Val-Ser-Leu-Lys-Cys-Thr-Asp-Leu-Gly-Asn-Ala-Thr-Asn-Thr-Asn-Ser-Ser-Asn-Thr-Asn-Ser-Ser-Ser-Gly-Glu-Met-Met-Met-Glu-Lys-Gly-Glu-Ile-Lys-Asn-Cys-Ser-Phe-Asn-Ile-Ser-Thr-Ser-Ile-Arg-Gly-Lys-Val-Gln-Lys-

acides aminés 197-201 compris, par exemple, Leu-Asp-Ile-Ile-Pro-Ile-Asp-Asn-Asp-Thr-Thr-

acides aminés 239-294 compris, par exemple, Lys-Cys-Asn-Asn-Lys-Thr-Phe-Asn-Gly-Thr-Gly-Pro-Cys-Thr-Asn-Val-Ser-Thr-Val-Gln-Cys-Thr-His-Gly-Ile-Arg-Pro-Val-Val-Ser-Thr-Gln-Leu-Leu-Leu-Asn-Gly-Ser-Leu-Ala-Glu-Glu-Glu-Val-Val-Ile-Arg-Ser-Ala-Asn-Phe-Thr-Asp-Asn-Ala-Lys-

acides aminés 300-327 compris, par exemple, Leu-Asn-Gln-Ser-Val-Glu-Ile-Asn-Cys-Thr-Arg-Pro-Asn-Asn-Asn-Thr-Arg-Lys-Ser-Ile-Arg-Ile-Gln-Arg-Gly-Pro-Gly-Arg-

acides aminés 334-381 compris, par exemple, Lys-Ile-Gly-Asn-Met-Arg-Gln-Ala-His-Cys-Asn-Ile-Ser-Arg-Ala-Lys-Trp-Asn-Ala-Thr-Leu-Lys-Gln-Ile-Ala-Ser-Lys-Leu-Arg-Glu-Gln-Phe-Gly-Asn-Asn-Lys-Thr-Ile-Ile-Phe-Lys-Gln-Ser-Ser-Gly-Gly-Asp-Pro-

acides aminés 397-424 compris, par exemple, Cys-Asn-Ser-Thr-Gln-Leu-Phe-Asn-Ser-Thr-Trp-Phe-Asn-Ser-Thr-Trp-Ser-Thr-Glu-Gly-Ser-Asn-Asn-Thr-Glu-Gly-Ser-Asp-

acides aminés 466-500 compris, par exemple, Leu-Thr-Arg-Asp-Gly-Gly-Asn-Asn-Asn-Asn-Gly-Ser-Glu-Ile-Phe-Arg-Pro-Gly-Gly-Gly-Asp-Met-Arg-Asp-Asn-Trp-Arg-Ser-Glu-Leu-Tyr-Lys-Tyr-Lys-Val-

acides aminés 621-630 compris, par exemple, Pro-Trp-Asn-Ala-Ser-Trp-Ser-Asn-Lys-Ser-

8. Procédé pour la préparation d'un peptide purifié selon l'une quelconque des revendications 3 ou 7 caractérisé en ce que le peptide récupéré est un peptide portant un épitope et plus particulièrement, en ce que ce peptide est un peptide immunogène.

9. Procédé pour la préparation d'un peptide purifié selon la revendication 8 caractérisé en ce que le peptide récupéré est mis ensuite sous forme oligomère, plus particulièrement, en oligomère immunogène, soluble dans l'eau,

10. Procédé pour la préparation d'un polypeptide hybride comprenant un peptide obtenu selon l'une quelconque des revendications 3, 4, 7 ou 8 et ayant aux extrémités des N-terminal ou C-terminal, lequel polypeptide est lié par une liaison covalente aux acides aminés autres que ceux qui leur sont normalement associés dans le gp110 du LAV lesquelles acides aminés cités en dernier sont, soit libres, soit font partie d'une autre séquence polypeptidique, plus particulièrement d'une séquence ayant une taille suffisante pour amener une augmentation de l'immunogénicité

11. Procédé pour la préparation d'une composition de peptides caractérisée en ce qu'elle est préparée par mélange de 2 peptides au moins selon l'une quelconque des revendications de 1 à 8.

12. Procédé pour la préparation d'une composition immunogène comprenant un peptide purifié obtenu selon la revendication 1, 2, 7 ou 8 ou un oligomère purifié selon la revendication 9 ou un polypeptide hybride selon la revendication 10, mélangé avec un vecteur pharmaceutique approprié.

13. Procédé de préparation d'une séquence ADN caractérisée en ce que :
    - l'ADN entier correspondant au génôme complet des espèces de LAV est clivé par digestion avec un enzyme de restriction approprié.
    - le fragment significatif codant pour un peptide tel qu'obtenu selon l'une queconque des revendication de 1 à 8, est récupéré.

14. Procédé pour la préparation d'une séquence d'ADN selon la revendication 13, caractérisé en ce que la séquence d'ADN est insérée dans un vecteur.

15. Procédé pour la synthèse chimique d'un peptide ou d'un polypeptide selon l'une des revendications de 1 à 8, caractérisé en ce que :
    - sont condensés, soit chacun des acides aminés successifs par paire, dans l'ordre approprié, soit des fragments peptidiques successifs disponibles ou formé préalablement et comprenant déjà plusieurs résidus aminoacylés dans l'ordre approprié respectivement, tous les groupes réactifs, autres que les groupes carboxyle ou amino, qui participeront à la formation de la liaison peptidique, étant protégés,
    - les groupes carboxyle sont activés, si possible, avant la formation de la liaison poypeptidique,
    - le peptide ou le polypeptide formé est récupéré.

16. Procédé pour le dignostic in vitro de la présence des anticorps anti-LAV dans des milieux biologiques caractérisé en ce qu'il comprend la mise en contact dudit fluide biologique avec le produit de l'une quelconque des revendications de 1 à 8 dans des conditions permettant la formation d'un complexe entre lesdits anticorps et ledit produit, la détection dudit complexe étant l'indicateur de la présence desdits anticorps dans ledit fluide biologique.

17. Procédé pour la production d'anticorps monoclonaux dirigés contre le peptide purifié tel qu'obtenu selon l'une quelconque des revendications de 1 à 9, comprenant les étapes suivantes :
    - fusion des cellules de myelome, obtenues d'un animal préalablement immunisé par ce peptide purifié, avec des splenocytes pour obtenir des hybridomes;
    - sélection des hybridomes sécréteurs des anticorps spécifiques pour le peptide purifié;
    - récupération des anticorps dirigés contre le peptide purifié.

18. Procédé pour la production d'anticorps caractérisé par l'immunisation d'un animal avec un peptide purifié, tel qu'obtenu selon les revendications de 1 à 9, et par la récupèration des anticorps reconnus par le peptide purifié selon l'une quelconque des revendications de 1 à 9.

19. Utilisation des anticorps selon la revendication 17 ou 18, pour l'identification de la glycoprotéine d'enveloppe du virus LAV ou même pour la détermination de la proportion relative de cet antigène dans l'échantillon biologique contenant le LAV ou des virus apparentés.

FIG.1.

5′ ⟵ ——————————————————————————————————————— 3′

H + H − H     |     +     H + H     λJ19

H   B   XK   SH    pLAV13
Bg     Bg   Bg

H + H − H     −     H     +     H + H     λJ81

HS   H   P   H      K   K   R     R Sa    K       H    XK   SH    λJ19
   BB     Bg            Bg     Bg    B   Bg   Bg

0    1    2    3    4    5    6    7    8    9   9.2 kb

R US                                           U3 R

H = Hind III    K = Kpn I
S = Sac I      R = EcoRI
B = Bam HI    Sa = Sal I
P = Pst I      X = Xho I

Bg = Bgl II

FIG.2.

EP 0 387 914 B1

# FIG. 3.

EP 0 387 914 B1

Hin.

CGTCTCTCTCGTTAGACCAGATTTCGAGCCTGCCGACCTCTCTGGCTAACTAGCGAACCCACTCCTTAAGCCTCAATAAAGCTTGCCTTGAGTGCCTTCAACTAGTGTGTGCCCGTCTGTTGT
100

GTCACTCTGGTAACTAGAGATCCCTCAGACCCCTTTTAGTCAGTCGTGGAAAATCTCTAGCAGTGCCGCCCCGAACAGCGCACTTCAAAGCCGAAAGGGCAAACCAGAGCAGCTCTCTCCACGCAG
200

GAG → LeuAlaGluAlaArgArgArgGlu[Met]GlyAlaArgAlaSerValLeuSer
GACTCGGCTTGCTGAAGCGCGCACCGCAAGAGCGGAGCGGAGCGCCACTCGTGAGTACGCCAAAAATTTTGACTAGCGGACGCTAGAAGGACAGAGATCGGTCGCGACAGCCGTCAGTATTAA
300

GlyGlyGluLeuAspArgTrpGluLysIleArgLeuArgProGlyGlyLysLysLysTyrLysLeuLysHisIleValTrpAlaSerArgGluLeuGluArgPheAlaValAsnProGly
CCCGCGCCAGAATTAGATCGATGGCAAAAAATTCCGGTTAAGCCCACGGGCAAAGAAAAAATATAAATTAAAACATATAGTATGGGCAAGCAGGGAGCTAGAACGATTCGCAGTTAATCCTC
400

LeuLeuGluThrSerGluGlyCysArgGlnIleLeuGlyGlnLeuGlnProSerLeuGlnThrGlySerGluGluLeuArgSerLeuTyrAsnThrValAlaThrLeuTyrCysValHis
GCCTGTTAGAAACATCAGAAGGCTGTAGACAAATACTCGGACAGCTACAACCATCCCTTCAGACAGGATCAGAAGAACTTAGATCATTATATAATACAGTAGCAACCCTCTATTGTGTGC
500                                                                              600

GlnArgIleGluIleLysAspThrLysGluAlaLeuAspLysIleGluGluGluGlnAsnLysSerLysLysLysAlaGlnGlnAlaAlaAlaAspThrGlyHisSerSerGlnValSer
ATCAAAGGATAGAGATAAAAGACACCAAGGAAGCTTTAGACAAGATAGAGGAAGAGCAAAACAAAAGTAAGAAAAAAGCACAGCAAGCAGCAGCTGACACGACACAGCCAGCCAGGTCA
700

GlnAsnTyr[ProIleValGlnAsnIleGlnGlyGlnMetValHisGlnAlaIle]SerProArgThrLeuAsnAlaTrpValLysValValGluGluLysAlaPheSerProGluValIle
GCCAAAATTACCCTATAGTGCAGAACATCCAGGGGCAAATGGTACATCAGGCCATATCACCTAGAACTTTAAATGCATGGGTAAAAGTAGTAGAAGAGAAGGCTTTCAGCCCAGAAGTGA
800

ProMetPheSerAlaLeuSerGluGlyAlaThrProGlnAspLeuAsnThrMetLeuAsnThrValGlyGlyHisGlnAlaAlaMetGlnMetLeuLysGluThrIleAsnGluGluAla
TACCCATGTTTTCAGCATTATCAGAAGGAGCCACCCCACAAGATTTAAACACCATGCTAAACACAGTGGGGCGGACATCAAGCAGCCATGCAAATGTTAAAAGAGACCATCAATGAGGAAG
900

AlaGluTrpAspArgValHisProValHisAlaGlyProIleAlaProGlyGlnMetArgGluProArgGlySerAspIleAlaGlyThrThrSerThrLeuGlnGluGlnIleGlyTrp
CTGCAGAATGGGATAGAGTGCATCCAGTGCATGCAGGGCCCTATTGCACCAGGCCAGATGAGAGAACAACCAACGGGAAGTGACATACCAGGAACTACTAGTACCCTTCAGGAACAAATAGGAT
1000

MetThrAsnAsnProProIleProValGlyGluIleTyrLysArgTrpIleIleLeuGlyLeuAsnLysIleValArgMetTyrSerProThrSerIleLeuAspIleArgGlnGlyPro
GGATGACAAATAATCCACCTATCCCAGTAGGAGAAATTTATAAAAGATGGATAATCCTGGGATTAAATAAAATAGTAAGAATGTATAGCCCTACCAGCATTCTGGACATAAGACAACCAG
1100                                                                             1200

LysGluProPheArgAspTyrValAspArgPheTyrLysThrLeuArgAlaGluGlnAlaSerGlnGluValLysAsnTrpMetThrGluThrLeuLeuValGlnAsnAlaAsnProAsp
CAAAAGAACCCTTTAGAGACTATGTAGACCCGTTCTATAAAACTCTAAGAGCCGAGCAAGCTTCACAGGAGGTAAAAAATTGGATGACAGAAACCTTGTTGGTCCAAAATGCGAACCCAG
1300

CysLysThrIleLeuLysAlaLeuGlyProAlaAlaThrLeuGluGluMetMetThrAlaCysGlnGlyValGlyGlyProGlyHisLysAlaArgValLeuAlaGluAlaMetSerGln
ATTGTAAGACTATTTTAAAAGCATTGGGACCAGCAGCTACACTAGAAGAAATGATGACAGCATGTCAGGCAGTGGCGAGGACCCGGCCATAAGGCAAGAGTTTTGGCTGAAGCAATGAGCC
1400

ValThrAsnSerAlaThrIleMetMetGlnArgGlyAsnPheArgAsnGlnArgLysIleValLysCysPheAsnCysGlyLysGluGlyHisIleAlaArgAsnCysArgAlaProArg
AAGTAACAAATTCAGCTACCATAATGATGCAAAGAGGCAATTTTAGGAACCAAAGAAAGATTGTTAAGTGTTTCAATTGTGGCAAAGAAGGGCCACATAGCCAGAAATTGCAGGGCCCCTA
1500

POL → PhePheArgGluAspLeuAlaPheLeuGlnGlyLysAlaArgGluPheSer
LysLysGlyCysTrpLysCysGlyLysGluGlyHisGlnMetLysAspCysThrGluArgGlnAlaAsnPheLeuGlyLysIleTrpProSerTyrLysGlyArgProGlyAsnPheLeu
GGAAAAAGGGCTGTTGGAAATGTGGAAAGGAAGGACACCAAATGAAAGATTGTACTGAGAGACAGGCTAATTTTTTAGGGAAGATCTGGCCTTCCTACAAGGGAAGGCCAGGGAATTTTC
1600

SerGluGlnThrArgAlaAsnSerProThrArgArgGluLeuGlnValTrpGlyArgAspAsnAsnSerLeuSerGluAlaGlyAlaAspArgGlnGlyThrValSerPheAsnPhePro
GlnSerArgProGluProThrAlaProProGluGluSerPheArgSerGlyValGluThrThrThrProSerGlnLysGlnGluProIleAspLysGluLeuTyrProLeuThrSerLeu
TTCAGAGCAGACCAGAGCCAACAGCCCCCACCAGAAGAGAGCTTCAGGTCTGGGGTAGAGACAACAACTCCCTCTCAGAAGCAGCAGCCCGATAGACAAGCAACTGTATCCTTTAACTTCCC
1700                                                                             1800

GlnIleThrLeuTrpGlnArgProLeuValThrIleLysIleGlyGlyGlnLeuLysGluAlaLeuLeuAspThrGlyAlaAspAspThrValLeuGluGluMetSerLeuProGlyArg
ArgSerLeuPheGlyAsnAspProSerSerGln ●
TCAGATCACTCTTTGGCAACGACCCCTCGTCACAATAAAGATAGGGGGGCAACTAAAGGAAGCTCTATTAGATACAGGAGCACATGATACAGTATTAGAAGAAATGAGTTTGCCAGGAAG
1900

FIG. 4a

FIG. 1 b

TrpLysProLys|Met|IleGlyGlyIleGlyGlyPheIleLysValArgGlnTyrAspGlnIleLeuIleGluIleCysGlyHisLysAlaIleGlyThrValLeuValGlyProThrPro
ATGGAAACCAAAAATGATAGCGGGAATTGGAGCGTTTTATCAAAGTAAGACAGTATGATCAGATACTCATAGAAATCTGTGGACATAAAGCTATAGGTACAGTATTAGTAGGACCTACACC
                                                                        2000

ValAsnIleIleGlyArgAsnLeuLeuThrGlnIleGlyCysThrLeuAsnPheProIleSerProIleGluThrValProValLysLeuLysProGlyMetAspGlyProLysValLys
TGTCAACATAATTGGAAGAAATCTGTTGACTCAGATTGGTTGCACTTTAAATTTTCCCATTAGTCCTATTGAAACTGTACCAGTAAAATTAAAGCCAGGAATGGATGGCCCAAAAGTTAA
                                                                        2100

GlnTrpProLeuThrGluGluLysIleLysAlaLeuValGluIleCysThrGluMetGluLysGluGlyLysIleSerLysIleGlyProGluAsnProTyrAsnThrProValPheAla
ACAATGGCCATTGACAGAAGAAAAAATAAAAGCATTAGTAGAAATTTGTACACAAATGGAAAAGGAAGGGAAAATTTCAAAAATTGGGCCTGAAAATCCATACAATACTCCAGTATTTGC
                                                                        2200

IleLysLysLysAspSerThrLysTrpArgLysLeuValAspPheArgGluLeuAsnLysArgThrGlnAspPheTrpGluValGlnLeuGlyIleProHisProAlaGlyLeuLysLys
CATAAAGAAAAAACACAGTACTAAATGGAGAAAATTAGTAGATTTCAGAGAACTTAATAACAGAACTCAAGACTTCTGGGAAGTTCAATTAGGAATACCACATCCCGCAGGGTTAAAAAA
                                                                        2300

LysLysSerValThrValLeuAspValGlyAspAlaTyrPheSerValProLeuAspGluAspPheArgLysTyrThrAlaPheThrIleProSerIleAsnAsnGluThrProGlyIle
GAAAAAATCAGTAACAGTACTGGATGTGGGTGATGCATATTTTTCAGTTCCCTTAGATGAAGACTTCAGGAAGTATACTGCATTTACCATACCTAGTATAAACAATGAGACACCAGGGAT
                                                                        2400

ArgTyrGlnTyrAsnValLeuProGlnGlyTrpLysGlySerProAlaIlePheGlnSerSerMetThrLysIleLeuGluProPheArgLysGlnAsnProAspIleValIleTyrGln
TAGATATCAGTACAATGTGCTTCCACAGGGATGGAAAGGATCACCAGCAATATTCCAAAGTAGCATGACAAAAATCTTAGAGCCCTTTTAGAAAACAAAATCCAGACATAGTTATCTATCA
                                                                        2500

TyrMetAspAspLeuTyrValGlySerAspLeuGluIleGlyGlnHisArgThrLysIleGluGluLeuArgGlnHisLeuLeuArgTrpGlyLeuThrThrProAspLysLysHisGln
ATACATGGATGATTTGTATGTAGGATCTGACTTAGAAATACGGCAGCATAGAACAAAAATAGAGGAGCTGAGACAACATCTGTTGACGTGGGGACTTACCACACCAGACAAAAAACATCA
                                                                        2600

LysGluProProPheLeuTrpMetGlyTyrGluLeuHisProAspLysTrpThrValGlnProIleValLeuProGluLysAspSerTrpThrValAsnAspIleGlnLysLeuValGly
GAAAGAACCTCCATTCCTTTGGATGGGTTATGAACTCCATCCTGATAAATGGACAGTACAGCCTATAGTGCTGCCAGAAAAAGACAGCTGGACTGTCAATGACATACAGAAGTTAGTGGG
                                                                        2700

LysLeuAsnTrpAlaSerGlnIleTyrProGlyIleLysValArgGlnLeuCysLysLeuLeuArgGlyThrLysAlaLeuThrGluValIleProLeuThrGluGluAlaGluLeuGlu
AAAATTGAATTGGGCAAGTCAGATTTACCCAGGGATTAAAGTAAGGCAATTATGTAAACTCCTTAGAGGAACCAAAGCACTAACAGAAGTAATACCACTAACAGAAGAAGCAGAGCTAGA
                                                                        2800

LeuAlaGluAsnArgGluIleLeuLysGluProValHisGlyValTyrTyrAspProSerLysAspLeuIleAlaGluIleGlnLysGlnGlyGlnGlyGlnTrpThrTyrGlnIleTyr
ACTGGCAGAAAACAGAGAGATTCTAAAAGAACCAGTACATGGAGTGTATTATGACCCATCAAAAGACTTAATAGCAGAAATACAGAAGCAGGGGCAAGGCCAATGGACATATCAAATTTA
                                                                        2900                                                                3000

GlnGluProPheLysAsnLeuLysThrGlyLysTyrAlaArgThrArgGlyAlaHisThrAsnAspValLysGlnLeuThrGluAlaValGlnLysIleThrThrGluSerIleValIle
TCAAGAGCCATTTAAAAATCTGAAAACAGGAAAATATGCAAGAACGAGGGGTGCCCACACTAATGATGTAAAACAATTAACAGAGGCAGTGCAAAAAATAACCACAGAAAGCATAGTAAT
                                                                        3100

TrpGlyLysThrProLysPheLysLeuProIleGlnLysGluThrTrpGluThrTrpTrpThrGluTyrTrpGlnAlaThrTrpIleProGluTrpGluPheValAsnThrProProLeu
ATGGGGAAAGACTCCTAAATTTAAACTACCCATACAAAAGGAAACATGGGAAACATGGTGGACAGAGTATTGGCAAGCCACCTGGATTCCTGAGTGGGAGTTTGTCAATACCCCTCCTTT
                                                                        3200

ValLysLeuTrpTyrGlnLeuGluLysGluProIleValGlyAlaGluThrPheTyrValAspGlyAlaAlaSerArgGluThrLysLeuGlyLysAlaGlyTyrValThrAsnArgGly
AGTGAAATTATGGTACCAGTTAGAGAAAGAACCCATAGTAGGAGCAGAAACGTTCTATGTAGATGGGGCAGCTAGCAGGGAGACTAAATTAGGAAAAGCAGGATATGTTACTAATAGAGG
                                                                        3300

ArgGlnLysValValThrLeuThrAspThrThrAsnGlnLysThrGluLeuGlnAlaIleHisLeuAlaLeuGlnAspSerGlyLeuGluValAsnIleValThrAspSerGlnTyrAla
AAGACAAAAAGTTGTCACCCTAACTGACACAACAAATCAGAAGACTGAGTTACAAGCAATTCATCTAGCTTTGCAGGATTCCGGGATTAGAAGTAAATATAGTAACAGACTCACAATATGC
                                                                        3400                                                                3500                                                                3600

LeuGlyIleIleGlnAlaGlnProAspLysSerG..uSerGluLeuValAsnGlnIleIleGluGlnLeuIleLysI  luLysValTyrLeuAlaTrpValProAlaHisLysGlyIle
ATTAGGAATCATTCAACCACAACCAGATAAAAGTGAATCAGAGTTAGTCAATCAAATAATAGAGCAGTTAATAAAAAAGGAAAAGGTCTATCTCGGCATGGGTACCAGCACACAAAGCAAT
3700

GlyGlyAsnGluGlnValAspLysLeuValSerAlaGlyIleArgLysValLeuPheLeuAspGlyIleAspLysAlaGlnAspGluHisGluLysTyrHisSerAsnTrpArgAlaMet
TGGAGGAAATGAACAAGTACATAAATTAGTCAGTGCTGGAATCAGGAAAGTACTATTTTTAGCATGGAATAGATAAGGCCCAAGATGAACATGACAAATATCACAGTAATTGGAGAGCAAT
3800

AlaSerAspPheAsnLeuProProValValAlaLysGluIleValAlaSerCysAspLysCysGlnLeuLysGlyGluAlaMetHisGlyGlnValAspCysSerProGlyIleTrpGln
GGCTAGTGATTTTAACCTGCCACCTGTAGTAGCCAAAAGAAATAGTAGCCAGCTGTCATAAATGTCAGCCTAAAAGGAGAAGCCATGCATGGACAAGTAGACTGTAGTCCAGGAATATGGCA
3900

LeuAspCysThrHisLeuGluGlyLysValIleLeuValAlaValHisValAlaSerGlyTyrIleGluAlaGluValIleProAlaGluThrGlyGlnGluThrAlaTyrPheLeuLeu
ACTACATTGTACACATTTACAAGGAAAAGTTATCCTGGTAGCAGTTCATGTAGCCAGTGGATATATAGAAGCAGAAGTTATTCCAGCAGAAACACGGCACGAAACAGCATACTTTCTTTT
4000

LysLeuAlaGlyArgTrpProValLysThrIleHisThrAspAsnGlySerAsnPheThrSerThrThrValLysAlaAlaCysTrpTrpAlaGlyIleLysLysGlnGluPheGlyIlePro
AAAATTAGCAGGAAGATCGCCAGTAAAAACAATACATACAGACAATGGCAGCAATTTCACCAGTACTACGGTTAAGGCCGCCTGTTGGTGGCGGGAATCAACCAGGAATTTGGAATTCC
4100                                                                          4200

TyrAsnProGlnSerGlnGlyValValGluSerMetAsnLysGluLeuLysLysIleIleGlyGlnValArgAspGlnAlaGluHisLeuLysThrAlaValGlnMetAlaValPheIle
CTACAATCCCCAAAGTCAAGGAGTAGTAGAATCTATGAATAAAGAATTAAAGAAAATTATAGGCCAGGTAAGAGATCAGGCTGAACATCTTAAGCACAGCAGTACAAATGCCAGTATTCAT
4300

HisAsnPheLysArgLysGlyGlyIleGlyGlyTyrSerAlaGlyGluArgIleValAspIleIleAlaThrAspIleGlnThrLysGluLeuGlnLysGlnIleThrLysIleGlnAsn
CCACAATTTTAAAAGAAAAGCGGGGATTGGGGGGTACAGTGCAGCGGGAAAGAATAGTAGACATAATAGCAACAGACATACAAACTAAAGAATTACAAAAACAAATTACAAAAAATTCAAAA
4400

PheArgValTyrTyrArgAspSerArgAspProLeuTrpLysGlyProAlaLysLeuLeuTrpLysGlyGluGlyAlaValValIleGlnAspAsnSerAspIleLysValValProArg
                                                                                                      ORF Q ~ CysGlnGlu
TTTTCGGGTTTATTACAGGCACACCAGAGATCCACTTTGGAAAGGACCACCAAAGCTCCTCTCTCGAAAGGTGAAGGCCCAGTAGTAATACAAGATAATAGTGACATAAAAGTAGTGCCAAG
4500

ArgLysAlaLysIleIleArgAspTyrGlyLysGlnMetAlaGlyAspAspCysValAlaSerArgGlnAspGluAsp •
GluLysGlnArgSerLeuGlyIleMetGluAsnArgTrpGlnValMetIleValTrpGlnValAspArgMetArgIleArgThrTrpLysSerLeuValLysHisHisMetTyrValSer
AAGAAAGCCAAAGATCATTAGGGATTATGGAAAACAGATGGCAGGTCATGATTGTGTGGCAAGTAGACAGGATGAGGATTAGAACATGGAAAAGTTTAGTAAAACACCATATGTATGTTT
4600

GlyLysAlaArgGlyTrpPheTyrArgHisHisTyrGluSerProHisProArgIleSerSerGluValHisIleProLeuGlyAspAlaArgLeuValIleThrThrTyrTrpGlyLeu
CACGGAAAGCCTAGGGGATCGTTTTATAGACATCACTATGAAGCCCCTCATCCAAGAATAAGTTCAGAAGTACACATCCCACTAGGGGATGCTAGATTGGTAATAACAACATATTGGGGTC
4700                                                                                             4800

HisThrGlyGluArgAspTrpHisLeuGlyGlnGlyValSerIleGluTrpArgLysLysArgTyrSerThrGlnValAspProGluLeuAlaAspGlnLeuIleHisLeuTyrTyrPhe
TGCATACAGGAGAAAGAGACTGGCATCTGGGTCAGGGAGTCTCCATAGAATGGAGGAAAAAGACATATAGCACACAAGTAGACCCTGAACTAGCAGACCAACTAATTCATCTGTATTACT
4900

AspCysPheSerAspSerAlaIleArgLysAlaLeuLeuGlyHisIleValSerProArgCysGluTyrGlnAlaGlyHisAsnLysValGlySerLeuGlnTyrLeuAlaLeuAlaAla
TTGACTGTTTTTCAGACTCTGCTATAAGAAAGCCCTTATTAGGACATATAGTTAGCCCTAGGTGTGAATATCAAGCAGGACATAACAAGGTAGGATCTCTACAATACTTGGCACTAGCAG
5000

LeuIleThrProLysLysIleLysProProLeuProSerValThrLysLeuThrGluAspArgTrpAsnLysProGlnLysThrLysGlyHisArgGlySerHisThrMetAsnGlyHis
CATTAATAACACCAAAAAAGCATAAAGCCACCTTTGCCTAGTGTTACGAAACTGACACAGGATAGATGGAACAAGCCCCAGAAGACCAAGGCCCACAGAGGGACCCACACAATGAATGGAC
5100

ACTAGAGCTTTTAGAGGAGCCTTAAGCAATGAAGCTGTTAGACATTTTCCTAGGATTTGGCTCCATGGCCTTAGGGCAACATATCTATGAAACTTATGCGGATACTTGGGCACGAGTCGAAGC
5200

CATAATAAGAATTCTGCAACAACTGCTGTTTATCCATTTCAGAATTGGGTGTCGACATAGCAGAATAGGCGTTACTCAACAGAGCAGAGCCAAGAAATGGAGCCAGTAGATCCTAGACTAG
5300                                                                          5400

ACCCCTGGAAGCATCCAGGAAGTCAGCCTAAAACTGCTTGTACCACTTCCTATTGTAAAAAGTGTTGCTTTCATTGCCAAGTTTGTTTCACAACAAAAGCCTTAGGCATCTCCTATGCCA
5500

GGAAGAAGCGGAGACAGCGACGAAGACCTCCTCAAGGCAGTCAGACTCATCAAGTTTCTCTATCAAAGCAGTAAGTAGTACATGTAATCCAACCTATACAAATAGCAATAGCACCATTAG
5600

FIG. 4 c

ENV ► LysGluGlnLysThr
TAGTAGCAATAATAATAGCCAATAGTTGTCTCGTCCATAGTAATCATAGAATATAGGAAAATATTAAGACAAAGAAAAATACACACGTTAATTCATACACTAATACAAAGACCACAACACA

5700

ValAlaMetArgValLysGluLysTyrGlnHisLeuTrpArgTrpGlyTrpLysTrpGlyThrMetLeuLeuGlyIleLeuMetIleCysSerAlaThrGluLysLeuTrpValThrVal
GTCCCAATCACAGTCAAGGACAAATATCACCACTTGTCGGACATGGGCGGTGGAAATGGCGGCACCATGCTCCTTGGGATATTGATGATCTGTAGTGCTACAGAAAAATTCTCGGTCACAGTC

5800

TyrTyrGlyValProValTrpLysGluAlaThrThrThrLeuPheCysAlaSerAspAlaLysAlaTyrAspThrGluValHisAsnValTrpAlaThrHisAlaCysValProThrAsp
TATTATGGGGTACCTGTCTGCGAAGGAAGCAACCACCACTCTATTTTGTGCATCAGATGCTAAAGCATATGATACAGAGGTACATAATGTTTGGGCCACACATGCCTGTGTACCCACAGAC

5900                                                                                              6000

ProAsnProGlnGluValValLeuValAsnValThrGluAsnPheAsnMetTrpLysAsnAspMetValGluGlnMetHisGluAspIleIleSerLeuTrpAspGlnSerLeuLysPro
CCCAACCCACAAGAAGTAGTATTCGTAAATGTCACAGAAAATTTTAACATGTGGAAAAATGACATGGTAGAACAGATGCATGAGGATATAATCAGTTTATGGGATCAAAGCCTAAAGCCA

6100

CysValLysLeuThrProLeuCysValSerLeuLysCysThrAspLeuGlyAsnAlaThrAsnThrAsnSerSerAsnThrAsnSerSerSerGlyGluMetMetMetGluLysGlyGlu
TGTGTAAAATTAACCCCACTCTGTGTTAGTTTAAAGTGCACTGATTTGGGGAATGCTACTAATACCAATAGTAGTAATACCAATAGTAGTAGCGGGGAAATGATGATGGAGAAAGGAGAG

6200

IleLysAsnCysSerPheAsnIleSerThrSerIleArgGlyLysValGlnLysGluTyrAlaPhePheTyrLysLeuAspIleIleProIleAspAsnAspThrThrSerTyrThrLeu
ATAAAAAACTGCTCTTTCAATATCAGCCACAAGCCATAAGAGGTAAGGTCCAGAAACAATATGCCATTTTTTTTATAAACTTGCATATAATACCAATAGATAATGATACTACCAGCTATACGTTG

6300

ThrSerCysAsnThrSerValIleThrGlnAlaCysProLysValSerPheGluProIleProIleHisTyrCysAlaProAlaGlyPheAlaIleLeuLysCysAsnAsnLysThrPhe
ACAAGTTGTAACACCTCAGTCATTACACAGGCCCTGTCCAAAGGTATCCTTTCAGCCAATTCCCATACATTATTGTGCCCCGGCTGGTTTTGCGATTCTAAAATGTAATAATAAGACGTTC

6400

AsnGlyThrGlyProCysThrAsnValSerThrValGlnCysThrHisGlyIleArgProValValSerThrGlnLeuLeuLeuAsnGlySerLeuAlaGluGluGluValValIleArg
AATGGAACAGGACCATGTACAAATGTCAGCACAGTACAATGTACACATGGAATTAGCCCAGTAGTATCAACTCAACTGCTGTTGAATGGCAGTCTAGCAGAAGAAGAGGTAGTAATTAGA

6500                                                                                              6600

SerAlaAsnPheThrAspAsnAlaLysThrIleIleValGlnLeuAsnGlnSerValGluIleAsnCysThrArgProAsnAsnAsnThrArgLysSerIleArgIleGlnArgGlyPro
TCTGCCAATTTCACAGACAATGCTAAAACCATAATAGTACACCTGAACCAATCTGTAGAAATTAATTGTACAAGACCCAACAACAATACAAGAAAAAGTATCCGTATCCAGAGCGGACCA

6700

GlyArgAlaPheValThrIleGlyLysIleGlyAsnMetArgGlnAlaHisCysAsnIleSerArgAlaLysTrpAsnAlaThrLeuLysGlnIleAlaSerLysLeuArgGluGlnPhe
GGGAGAGCCATTTGTTACAATAGGAAAAATAGGAAATATGAGACAAGCACATTGTAACATTAGTAGAGCAAAATGGAATGCCACTTTAAAACAGATAGCTAGCAAATTAAGAGAACAATTT

6800

GlyAsnAsnLysThrIleIlePheLysGlnSerSerGlyGlyAspProGluIleValThrHisSerPheAsnCysGlyGlyGluPhePheTyrCysAsnSerThrGlnLeuPheAsnSer
GGAAATAATAAAACAATAATCTTTAAGCAATCCTCAGGAGGCGACCCAGAAATTGTAACCCACAGTTTTAATTGTGGAGGGGAATTTTTCTACTGTAATTCAACACAACTGTTTAATAGT

6900

ThrTrpPheAsnSerThrTrpSerThrGluGlySerAsnAsnThrGluGlySerAspThrIleThrLeuProCysArgIleLysGlnPheIleAsnMetTrpGlnGluValGlyLysAla
ACTTGGTTTAATAGTACTTGGAGTACTGAAGGGTCAAATAACACTGAAGGAAGTGACACAATCACACTCCCATGCAGAATAAAACAATTTATAAACATGTGCCAGGAACTAGCAAAACCA

7000

MetTyrAlaProProIleSerGlyGlnIleArgCysSerSerAsnIleThrGlyLeuLeuLeuThrArgAspGlyGlyAsnAsnAsnAsnGlySerGluIlePheArgProGlyGlyGly
ATGTATGCCCCTCCCATCAGCGGACAAATTAGATGTTCATCAAATATTACAGGGCTGCTATTAACAAGAGATGGTGGTAATAACAACAATGGGTCCGAGATCTTCAGACCTGGAGGAGGA

7100                                                                                              7200

AspMetArgAspAsnTrpArgSerGluLeuTyrLysTyrLysValValLysIleGluProLeuGlyValAlaProThrLysAlaLysArgArgValValGlnArgGluLysArgAlaVal
GATATGAGGGACAATTGGAGAAGTGAATTATATAAATATAAAGTAGTAAAAATTGAACCATTAGGAGTAGCACCCACCAAGGCAAAGACAAGACTGGTGCCAGAGAGAAAAAAGACCACTG

7300

GlyIleGlyAlaLeuPheLeuGlyPheLeuGlyAlaAlaGlySerThrMetGlyAlaArgSerMetThrLeuThrValGlnAlaArgGlnLeuLeuSerGlyIleValGlnGlnGlnAsn
GGAATAGCAGCGTTTCTTCCTTCGGTTCGTTCGGAGCAGCAGGAAGCACTATGGGCGCACGGTCAATGACGCTGACGGTACAGGCCAGACAATTATTGTCTCGGTATAGTCCACCAGCACGAAC
                        ·                   ·                   ·         7400      ·                   ·                   ·
AsnLeuLeuArgAlaIleGluAlaGlnGlnHisLeuLeuGlnLeuThrValTrpGlyIleLysGlnLeuGlnAlaArgIleLeuAlaValGluArgTyrLeuLysAspGlnGlnLeuLeu
AATTTGCTGAGCGCTATTGAGGCGCAACAGCATCTGTTGCAACTCACAGTCTGGGCCCATCAAGCAGCTCCAGCCAAGAATCCTCGCTGTCGAAAGATACCTAAAGGATCAACACCTCCTG
                        ·                   ·                   ·         7500      ·                   ·                   ·
GlyIleTrpGlyCysSerGlyLysLeuIleCysThrThrAlaValProTrpAsnAlaSerTrpSerAsnLysSerLeuGluGlnIleTrpAsnAsnMetThrTrpMetGluTrpAspArg
GGCATTTGGCGGTTGCTCTCGAAAACTCATTTGCACCGACTGCTGTGCCTTGGAATGCTAGTTCGAGTAATAAATCTCTGGAACAGATTTGGAATAACATGACGTGCATCGAGTCCCACACA
                        ·                   ·                   ·         7600      ·                   ·                   ·
GluIleAsnAsnTyrThrSerLeuIleHisSerLeuIleGluGluSerGlnAsnGlnGlnGluLysAsnGluGlnGluLeuLeuGluLeuAspLysTrpAlaSerLeuTrpAsnTrpPhe
GAAATTAACAATTACACAAGCTTAATACATTCCTTAATTCAAGAATCGCAAAACCAGCAAGAAAAGAATGAACAAGAATTATTGGAATTACATAAATGGGCAAGTTTGTGGAATTCGTTT
                        ·     7700      ·                   ·                   ·                   ·         7800
AsnIleThrAsnTrpLeuTrpTyrIleLysIlePheIleMetIleValGlyGlyLeuValGlyLeuArgIleValPheAlaValLeuSerIleValAsnArgValArgGlnGlyTyrSer
AACATAACAAATTGGCTGTGGTATATAAAAATATTCATAATGATAGTAGGAGGCTTGGTAGGTTTAAGAATAGTTTTTTGCTGTACTTTCTATAGTGAATAGAGTTAGGCAGGCGATATTCA
                        ·                   ·                   ·                   ·         7900      ·
ProLeuSerPheGlnThrHisLeuProThrProArgGlyProAspArgProGluGlyIleGluGluGluGlyGlyGluArgAspArgAspArgSerIleArgLeuValAsnGlySerLeu
CCATTATCGTTTCAGACCCACCTCCCAACCCCGAGGGGACCCGACAGGCCCGAAGGAATAGAAGAAGAAGGTGGAGAGAGAGACAGAGACAGATCCATTCGATTAGTCAACGGATCCTTA
                        ·                   ·                   ·                   ·         8000      ·
AlaLeuIleTrpAspAspLeuArgSerLeuCysLeuPheSerTyrHisArgLeuArgAspLeuLeuLeuIleValThrArgIleValGluLeuLeuGlyArgArgGlyTrpGluAlaLeu
GCACTTATCTGGGACGATCTGCGGAGCCTGTGCCTCTTCAGCTACCACCGCTTGACAGAGTTACTCTTGATTGTAACGAGGATTGTGGAACTTCTGGGACGGAGCGCGTGGGAAGCCCTC
                        ·                   ·                   ·         8100      ·                   ·
LysTyrTrpTrpAsnLeuLeuGlnTyrTrpSerGlnGluLeuLysAsnSerAlaValSerLeuLeuAsnAlaThrAlaIleAlaValAlaGluGlyThrAspArgValIleGluValVal
AAATATTGGTGGAATCTCCTACAGTATTGGAGTCAGGAACTAAAGAATAGTGCTGTTAGCTTGCTCAATGCCACAGCCATAGCAGTAGCTGAGGGGACAGATAGGGTTATAGAAGTAGTA
                        ·                   ·         8200      ·                   ·                   ·
GlnGlyAlaCysArgAlaIleArgHisIleProArgArgIleArgGlnGlyLeuGluArgIleLeuLeu •
                                    ORF F ⊢ AspArgAlaTrpLysGlyPheCysTyrLys Met GlyGlyLysTrpSerLysSerSerValValGlyTrpProThrVal
CAAGGAGCCTTGTAGAGCCTATTCGCCACATACCTAGAAGAATAAGACACGGCCTTGGAAAGGATTTTGCTATAAGATCGGCTGGCAACTGGTCAAAAAGTAGTCTGGTTCGATCGCCTACTGT
            8300      ·                   ·                   ·                   ·                   ·         8400
ArgGluArgMetArgArgAlaGluProAlaAlaAspGlyValGlyAlaAlaSerArgAspLeuGluLysHisGlyAlaIleThrSerSerAsnThrAlaAlaThrAsnAlaAlaCysAla
AACCGAAAGAATGAGACGAGCTGAGCCAGCACCAGATCGGGCTGGGAGCACCATCTCCGAGACCTGGAAAAACATGGAGCAATCACAAGTAGCAATACAGCAGCTACCAATGCTGCTTGTGCC
                ·                   ·                   ·                   ·                   ·         8500      ·
TrpLeuGluAlaGlnGluGluGluGluValGlyPheProValThrProGlnValProLeuArgProMetThrTyrLysAlaAlaValAspLeuSerHisPheLeuLysGluLysGlyGly
CTCGGCTAGAAGCACAAGAGGAGGAGGAGGTGCGGTTTTCCAGTCACACCTCAGGTACCCTTTAAGACCAATGACTTACAAGGCAGCTGTAGATCTTACCCACTTTTTAAAAGAAAAGGGGGG
                ·                   ·                   ·         8600      ·                   ·                   ·
LeuGluGlyLeuIleHisSerGlnArgArgGlnAspIleLeuAspLeuTrpIleTyrHisThrGlnGlyTyrPheProAspTrpGlnAsnTyrThrProGlyProGlyValArgTyrPro
ACTCGAAGGCCTAATTCACTCCCAACGAAGACAAGATATCCTTGATCTGTCGATCTACCACACACAAGGCTACTTCCCTCATTGGCAGAACTACACACCAGGGCCAGGCGTCAGATATCC
                ·                   ·                   ·                   ·         8700      ·
LeuThrPheGlyTrpCysTyrLysLeuValProValGluProAspLysValGluGluAlaAsnLysGlyGluAsnThrSerLeuLeuHisProValSerLeuHisGlyMetAspAspPro
ACTGACCTTTGGATCGTCCTACAAGCTAGTACCAGTTGAGCCAGATAAGGTAGAAGAGGCCAATAAAGGAGACAACACCAGCTTGTTACACCCTGTGAGCCTGCATGGAATGGATGACCCC
                ·                   ·         8800      ·                   ·                   ·
GluArgGluValLeuGluTrpArgPheAspSerArgLeuAlaPheHisHisValAlaArgGluLeuHisProGluTyrPheLysAsnCys •
TGACAGAGAAGTGTTACAGTGGACGTTTCACAGCCGCCTAGCATTTCATCACGTGCCCCGAGAGCTGCATCCGGAGTACTTCAAGAACTGCTGACATCGAGCTTGCTACAAGGGACTTTC
                ·         8900      ·                   ·                   ·                   ·         9000
CGCTGCGCCACTTTCCAGGGAGCCGTGCCCTGCCCGGCACTGCGGAGTGCCGAGCCCTCAGATGCTGCATATAAGCAGCTGCTTTTTGCCTGTACTGGCTCTCTCTGGTTAGACCAGATTT
GAGCCTGCGAGCTCTCTGGCTAACTAGGGAACCCACTGCTTA··GCCTCAATAAAGCTTGCCTTGAGTGCTTCA
                                    ·         Hind III ·                   ·                   ·         9100
                                            9190

FIG. 4 e

EP 0 387 914 B1

FIG. 5

FIG. 6

53